(19) **Europäisches Patentamt European Patent Office Office européen des brevets**

(11) **EP 3 195 849 B1**

(12) **EUROPEAN PATENT SPECIFICATION**

(45) Date of publication and mention of the grant of the patent:
**28.07.2021 Bulletin 2021/30**

(51) Int Cl.:
*A61K 8/19* (2006.01)   *A61K 8/29* (2006.01)
*A61K 8/27* (2006.01)   *A61K 8/02* (2006.01)
*A61Q 17/04* (2006.01)   *A61Q 19/00* (2006.01)
*A61K 8/89* (2006.01)

(21) Application number: **15843047.0**

(22) Date of filing: **15.09.2015**

(86) International application number:
**PCT/KR2015/009679**

(87) International publication number:
**WO 2016/043503 (24.03.2016 Gazette 2016/12)**

(54) **UV BLOCKING FUNCTIONAL COMPOSITE POWDER PREPARED BY DOUBLY COATING INORGANIC POWDER WITH INORGANIC UV BLOCKING AGENT AND ORGANIC UV ABSORBER, AND UV BLOCKING COSMETIC COMPOSITION USING SAME**

UV-BLOCKIERENDES FUNKTIONELLES VERBUNDPULVER, DAS DURCH DIE DOPPELTE BESCHICHTUNG VON ANORGANISCHEM PULVER MIT ANORGANISCHEM UV-BLOCKER UND ORGANISCHEM UV-ABSORBER HERGESTELLT WIRD, UND UV-BLOCKIERENDE, KOSMETISCHE ZUSAMMENSETZUNG MIT VERWENDUNG DAVON

POUDRE COMPOSITE FONCTIONNELLE BLOQUANT LES UV ÉLABORÉE PAR LE DOUBLE ENROBAGE D'UNE POUDRE INORGANIQUE AVEC UN AGENT BLOQUANT LES UV INORGANIQUE ET UN ABSORBEUR UV ORGANIQUE, ET COMPOSITION COSMÉTIQUE BLOQUANT LES UV ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.09.2014 KR 20140122357**
**25.02.2015 KR 20150026673**

(43) Date of publication of application:
**26.07.2017 Bulletin 2017/30**

(73) Proprietor: **Chemland. Co., Ltd.**
**Pyeongtaek-si, Gyeonggi-do 17701 (KR)**

(72) Inventors:
• **LEE, Beom Zoo**
**Seongnam-si**
**Gyeonggi-do 13540 (KR)**
• **JEONG, Soon Kyu**
**Goyang-si**
**Gyeonggi-do 10504 (KR)**
• **LEE, Seung Woo**
**Yongin-si**
**Gyeonggi-do 17091 (KR)**

• **HUR, Tak**
**Yongin-si**
**Gyeonggi-do 17035 (KR)**
• **KIM, Min Tae**
**Gunpo-si**
**Gyeonggi-do 15864 (KR)**
• **SHIN, Ji Woo**
**Seoul 08718 (KR)**
• **JEON, Sung Hee**
**Gunpo-si**
**Gyeonggi-do 15859 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
WO-A1-03/104319   WO-A2-02/22098
JP-A- 2012 121 810   KR-A- 20110 001 537
KR-A- 20110 062 501   KR-A- 20130 047 266
KR-A- 20140 046 507   KR-A- 20140 046 507

KR-B1- 101 062 430

- None

**Description**

**[TECHNICAL FIELD]**

The present invention relates to method for the preparation of a UV blocking functional composite powder prepared by doubly coating an inorganic powder with an inorganic UV blocking agent and an organic UV absorbent, and a UV blocking cosmetic composition using the same. More specifically, the present invention relates to a composite powder having excellent UV blocking functions without causing skin irritation by primarily coating an inorganic powder which is a coating base material with an inorganic UV blocking agent, secondarily coating the inorganic powder with an UV organic absorbent encapsulated into a liposome, and then tertiarily coating the inorganic powder with silicone oil, and a UV blocking cosmetic composition using the same.

**[BACKGROUND ART]**

Light is divided into visible light (400 to 760 nm), infrared light with a wavelength of 760 nm or higher, ultraviolet light with a wavelength of 400 nm or less, X-ray, γ-ray and the like. The ultraviolet light is divided into UVC (200 to 280 nm), UVB (280 to 320 nm) and UVA (320 to 400 nm) depending on the wavelength. UVC destroys DNA binding and causes skin cancer, but since it has a short wavelength to cause weak penetration, it is mostly absorbed in the ozone layer and the stratosphere (Yasuo, I., The mechanism of oryzanol activity and consideration to applicating efficiency in cosmetic, J. Fragrance 45: 92-97, 1980), and UVB inhibits the synthesis of nucleic acids and proteins in the skin and causes burns and skin erythema. In addition, UVA has only 1/1000 of energy compared to UVB, but the amount of light reaching the surface of the earth is 100 times of UVB and has been known to be responsible for skin pigmentation and aging (Sydney, H. D., Contact sensitization and photocontact sensitization of sunscreening agents. Physician's Guide to Sunscreens, 95-122, 1981).

KR 2013/0047266 relates to an illite composite powder for sunscreen functional cosmetics, the illite being used as a coating agent.

Recently, as the ozone layer protecting the ultraviolet rays has become thinner, and the ultraviolet ray index reaching the surface of the earth has increased, the interests of consumers in skin care are also increasing. Accordingly, UV blocking functional cosmetics containing UV blocking agents that protect skin from ultraviolet rays are gaining popularity from consumers, and the market size thereof shows an annual growth rate of 5% (Analysis report of cosmetics industry in 2013, Korea Health Industry Development Institute).

The ultraviolet blocking agents used in UV blocking functional cosmetics are divided into ultraviolet organic absorbents that absorb ultraviolet rays and convert energy into heat, waves, fluorescence, radicals, etc. to protect the skin and ultraviolet inorganic blocking agents that scatter ultraviolet rays. The ultraviolet organic absorbent has the advantage of having excellent absorbing power for ultraviolet ray A or ultraviolet ray B but has a disadvantage in that it can cause skin erythema and allergy as an ingredient which has not yet been tested for harmfulness to human body. In addition, the ultraviolet inorganic blocking agents are less harmful to the human body than the ultraviolet absorbents and have the ability to block a wide range of ultraviolet rays, which is advantageous.

These ultraviolet inorganic blocking agents and ultraviolet organic absorbents have advantages and disadvantages, and thus adding both ultraviolet blocking agents to cosmetics gives rise to excellent ultraviolet blocking functions. In the case of a cream formulation such as sunblock, the two ultraviolet blocking agents can be easily added, and SPF50, PA+++ sunblock products and the like are currently on the market. On the other hand, in the case of powder cosmetics, the inorganic UV blocking agents and organic absorbents should be coated on an inorganic powder such as talc or sericite, which is a basic ingredient, but there is a problem in that the binding between the inorganic powder and the blocking agent is difficult due to the nature of the formulation. Therefore, when adding 25% or more of the two ultraviolet blocking agents to the powder cosmetics, an aggregation between the powders may occur, which is problematic, and thus, unlike the sunblock, SPF 50, PA+++ powder cosmetics having an excellent ultraviolet blocking function have not been yet developed.

Therefore, there is a need to develop a composite powder which can be used as a power cosmetic and in which an ultraviolet blocking function is maximized without causing skin irritation, and a cosmetic composition using the same.

With respect to the present invention, doped titanium dioxide, a process for its preparation, a photocatalyst and an absorbent for UV radiation containing titanium dioxide, and purifying processes using titanium dioxide are disclosed in Korean Patent No. 10-0445543. However, it is mainly related to doping of the inorganic blocking agent for UV radiation containing titanium dioxide. Further, a composition for blocking of ultraviolet radiation comprising multi-layer encapsulated titanium dioxide and silica sol as an active ingredient and a manufacturing process thereof are disclosed in Korean Patent No. 10-0744945, but this is associated with coating titanium dioxide, zinc oxide or cerium oxide with silicone oil. On the other hand, a cosmetic composition for blocking UV light comprising dispersed $TiO_2$ with oil is disclosed in Korean Patent No. 10-1006343 but, it is associated with a preparation method for titanium dioxide oil dispersion by coating silicone oil with titanium dioxide/aluminum oxide, etc. and adding zirconium beads. However, a UV blocking functional composite powder of a structure prepared by primarily coating an inorganic powder such as sericite or talc with titanium dioxide or zinc oxide, which are inorganic UV blocking agents, secondarily coating the inorganic powder with ethylhexyl methoxycinnamate or butyl methoxydibenzoylmethane which are UV organic absorbents, and then tertiarily coating the inorganic powder with silicone oil, and a UV blocking cosmetic composition using the same are neither disclosed, suggested, nor taught in any of the patent literatures above.

**[DETAILED DESCRIPTION OF THE INVENTION]**

**[Technical Problem]**

Accordingly, one object of the present invention is to provide a method for preparing an organic-inorganic composite powder having an excellent UV blocking function without causing skin irritations by primarily coating an inorganic powder such as talc and sericite with titanium oxide or zinc oxide, which are inorganic UV blocking agents, secondarily coating the inorganic powder with ethylhexyl methoxycinnamate or butyl methoxydibenzoylmethane which are organic UV absorbents encapsulated into a liposome, and then tertiarily coating the inorganic powder with triethoxycaprylylsilane, which is silicone oil, to prevent the ultraviolet absorbents from being absorbed into the skin.

Another object of the present invention is to provide a UV blocking cosmetic composition containing the organic-inorganic composite powder, as an active ingredient, prepared by the method above.

**[Technical Solution]**

The objects of the present invention were achieved by the steps of preparing an inorganic-inorganic coating powder by primarily coating talc or sericite with titanium dioxide or zinc oxide; separately preparing an UV absorbent sol by encapsulating ethylhexyl methoxycinnamate or butyl methoxydibenzoylmethane in lecithin; preparing an organic-inorganic coating power by secondarily coating the inorganic-inorganic coating powder prepared in the step above, as a coating base with the UV absorbent sol, followed by tertiarily coating the powder with triethoxycaprylylsilane which is silicone oil; and measuring the presence or absence of coating of the organic-inorganic composite powder obtained in the step above, the content of the coating powder, ultraviolet protection function and skin irritation properties.

**[ADVANTAGEOUS EFFECTS]**

The organic-inorganic composite powder of the present invention maximizes the ultraviolet protection ability by simultaneously coating an inorganic powder with an UV inorganic blocking agent and an organic absorbent, has an effect of not causing skin irritation by encapsulating the organic absorbent, which can cause skin erythema, with hydrogenated lecithin, followed by coating the same, and provides an excellent effect of significantly increasing the value of the ultraviolet blocking agent in terms of function and quality when used as a UV blocking cosmetic composition.

**[BRIEF DESCRIPTION OF DRAWINGS]**

FIG. 1 is a diagram showing a process for preparing an inorganic-inorganic coating powder according to the present invention.
FIG. 2 shows images of the phase separation of pigment titanium dioxide-coated powder according to the present invention.
FIG. 3 shows images of the phase separation of nano-sized titanium dioxide-coated powder of according to the present invention.
FIG. 4 shows images of the phase separation of pigment zinc oxide-coated powder according to the present invention.
FIG. 5 shows images of the phase separation of nano-sized zinc oxide-coated powder of according to the present invention.
FIG. 6 shows images illustrating the transparency of the filtrate of pigment titanium dioxide-coated powder according to the present invention.
FIG. 7 shows images illustrating the transparency of the filtrate of nano-sized titanium dioxide-coated powder according to the present invention.

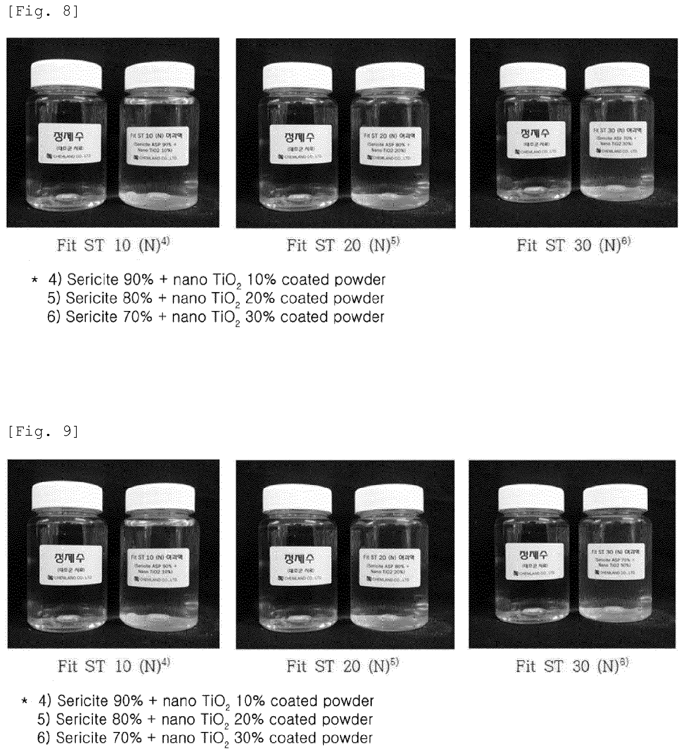

FIG. 8 shows images illustrating the transparency of the filtrate of pigment zinc oxide-coated powder according to the present invention.
FIG. 9 shows images illustrating the transparency of the filtrate of nano zinc oxide-coated powder according to the present invention.

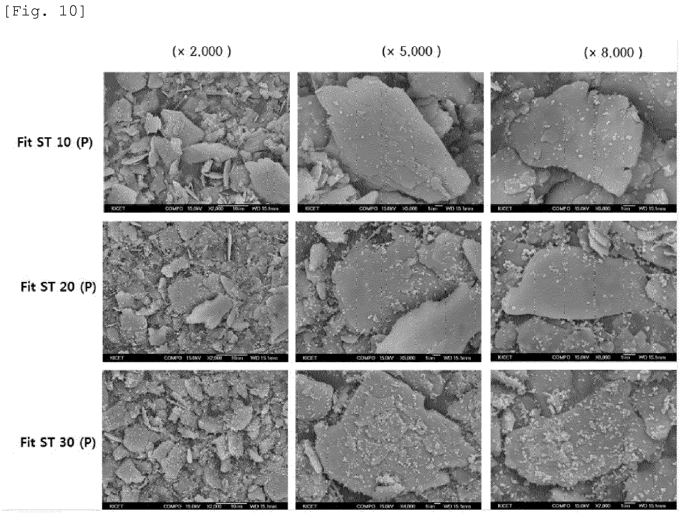

FIG. 10 shows FE-SEM images of pigment titanium dioxide-coated powder according to the present invention.
FIG. 11 shows FE-SEM images of nano-sized titanium dioxide-coated powder according to the present invention.
FIG. 12 shows FE-SEM images of pigment zinc oxide-coated powder according to the present invention.
FIG. 13 shows FE-SEM images of nano-sized zinc oxide-coated powder according to the present invention.
FIG. 14 is a diagram showing a process for preparing an ultraviolet organic absorbent sol according to the present invention.
FIG. 15 is a schematic diagram showing that the ultraviolet organic absorbent sol according to the present invention is encapsulated into hydrogenated lecithin.
FIG. 16 is a diagram showing a process for preparing an organic-inorganic coating powder according to the present invention.
FIG. 17 is a table showing the stimulus criteria for skin irritation clinical test according to the present invention.
FIG. 18 is a table showing the stimulus criteria for a skin irritation clinical test according to the present invention.
FIG. 19 is a table showing the SPF index of powder products containing the organic-inorganic coating powder according to the present invention.
FIG. 20 is a table showing the UVA PF index of powder products containing the organic-inorganic coating powder according to the present invention.

**[DETAILED DESCRIPTION OF THE EMBODIMENTS]**

The UV blocking functional composite powder of the present invention is prepared by a wet coating preparation process as described below.

**Step 1: Preparation of inorganic-inorganic powder (Primary Coating)**

(a) Preparing a coating base dispersion by adding 70 to 90% by weight of inorganic powder, as a coating base, based on the weight of purified water into 500 to 800% by weight of purified water at 75 to 80°C, followed dispersing and stirring the mixture for 25 to 30 minutes;

(b) Separately, preparing an inorganic blocking agent sol by dispersing 10 to 30% by weight of an UV inorganic blocking agent based on the weight of purified water in water and then pulverizing with a bead mill;

(c) Subjecting the inorganic blocking agent sol of step(b) to a primary wet coating by adding it to the coating base dispersion of step (a) and stirring for 25 to 30 minutes;

(d) Phase-separating the coating power and purified water and confirming the transparency thereof by allowing the coating solution to stand and cool at room temperature;

(e) Removing purified water from the coating solution, dehydrating and filtering the remaining coating powder, followed by drying the same at 105 to 110°C for 15 hours or more to make the water content to 1% or less; and

(F) Pulverizing the dried coating powder with an atomizer to prepare an UV blocking inorganic-inorganic powder of the present invention.

**Step 2: Preparation of organic-inorganic composite powder (Secondary and Tertiary Coating)**

(a-1) Adding and dispersing 1 to 5% by weight of butylene glycol, 0.1 to 1.0% by weight of tocopheryl acetate, and 1 to 10% by weight of hydrogenated lecithin based on the weight of purified water in 500 to 900 % by weight of purified water at 70 to 80°C, then adding, dispersing and liposomizing ethylhexyl methoxycinnamate, followed by treating the resultant twice with an ultra-high pressure emulsifier and cooling the same below 40°C at room temperature to prepare an ethylhexyl methoxycinnamate sol (Sol);

(a-2) Separately, adding and dispersing 1 to 5% by weight of butylene glycol, 0.1 to 1.0% by weight of tocopheryl acetate, and 1 to 10% by weight of hydrogenated lecithin based on the weight of purified water in 500 to 900% by weight of purified water at 70 to 80°C, and then adding, dispersing and liposomizing butyl methoxydibenzoylmethane dissolved in a mixture solution of $C_{12}$-$C_{15}$ alkyl benzoate, dipropylene glycol dibenzoate and PPG-15 stearyl ether benzoate, followed by cooling the resultant below 40°C at room temperature to prepare a butyl methoxydibenzoyl methane sol;

(b) Separately, adding 70 to 95% by weight of the inorganic-inorganic powder prepared in step 1, as a coating base, based on the weight of purified water to purified water at 75°C to 80°C and stirring the mixture for 20 to 30 minutes, following by adding thereto 0.1 to 5.0% by weight of magnesium metal salt and stirring for 10 to 20 minutes to prepare an inorganic-inorganic powder dispersion;

(c) Subjecting the ethylhexyl methoxycinnamate sol or butyl methoxydibenzoylmethane sol obtained in step 2-(a) to a wet secondary coating by adding the same to the inorganic-inorganic powder dispersion of step 2-(b) and stirring the mixture for 25 to 30 minutes;

(d) Phase-separating the coating powder from purified water and confirming the transparency thereof by allowing the coating solution to stand and cool at room temperature;

(e) Removing purified water from the coating solution, dehydrating and filtering the remaining coating powder, followed by drying it at 70°C to 80°C for 15 hours or more to make the water content to 1% or less, and then primarily pulverizing with an atomizer;

(f) Subjecting pulverized powder to surface-tertiary coating with 3% by weight of silicone oil; and

(g) Drying the tertiary-coated coating powder and secondarily pulverizing with an atomizer to prepare an ultraviolet protection organic-inorganic composite powder.

According to the present invention, the inorganic powder of step 1-(a) may be any one of talc, sericite, mica, silica, illite, kaolin and barium sulfate, and preferably, talc and sericite may be used. Sericite is an inorganic powder used as an extender pigment together with talc and mica in powder cosmetics. Talc has excellent spreadability and softness to skin and almost no elasticity, and thus, it shows a characteristic of maintaining a molding state with good tableting pressure when applied to two way cake and Powder Pact, which require tableting. Sericite, on the other hand, is a clay mineral which has poorer spreadability and softness than talc, but when a tableted product is rubbed with a puff, it helps to have a proper amount of the product smeared into the puff which then can be applied to the skin.

The UV blocking agent of step 1-(b) may use titanium dioxide ($TiO_2$) and zinc oxide (ZnO) in an amount of 10 to 30% by weight based on the weight of purified water. The formulations of titanium dioxide and zinc oxide may include a pigment inorganic blocking agent having a particle size of 0.25 to 0.30 $\mu$m, which used to impart whiteness to powder cosmetics, or a nano-sized inorganic blocking agent having a particle size of 30 to 50 nm, which is mainly used in sunscreen or the like.

The step of confirming the phase separation of the coating solutions in steps 1-(d) and 2-(e) is an important step for confirming the coating progress between the coating base and the UV blocking agent. If the coating process does not properly occur, the phase separation may not properly occur, or the coating base will exist in the upper layer.

Further, the magnesium metal salt in step 2-(b) is a cross-linking agent for crosslinking the inorganic-inorganic powder prepared in step 1 and the UV absorbent sol prepared in step 2-(a), and magnesium sulfate ($MgSO_4$) or anhydride ($XH_2O$) may be used.

The silicone oil of step 2-(g) may be methicone or triethoxycaprylylsilane (OTS), etc. However, in the case of methicone, in the present invention, hydrogen gas may be generated when an emulsifier is applied, thus, it was most preferable to use 3 to 5% by weight of OTS. Furthermore, in general, silicone oil for imparting water repellency may be coated by a dry process, but in the present invention, silicone oil may be coated on a base material by a wet process.

According to the present invention, in the case of the inorganic-inorganic coating powders of the present invention, it was confirmed through Experimental Examples 1 to 5 that when the phase-separation was confirmed after coating the inorganic powder with the UV inorganic blocking agents ($TiO_2$, ZnO) of different types and concentrations, the coating was properly carried out as the separation between the powder and coating solution was proceeded. As a result of the FE-SEM analysis, it was confirmed that the UV blocking agent applied as a coating agent was coated on the base material, and as a result of the EDS analysis, all the inorganic ingredients used as the base material and coating agents were confirmed, thereby confirming that the coating of the UV inorganic blocking agent was properly carried out which used the inorganic powder as a base material.

Further, through Experimental Example 6, when the UV blocking index of the inorganic-inorganic coating powder coated at different sizes and concentrations was measured by a non-clinical method, the UV blocking index of the composite powders, in which nano $TiO_2$ and ZnO were coated at a concentration of 30%, was measured to be the highest, and thus, this was selected as the base material for coating UV inorganic absorbents (OMC and BMDBM)

According to the present invention, in order to protect the disadvantages of the UV inorganic absorbent which can be harmful when made direct contact with the skin, a process of encapsulating the absorbent into a liposome was carried out. Then, the organic-inorganic composite powders (OMC and BMDBM coated powders) of the present invention, in which the organic absorbent sol encapsulated into a lecithin was coated onto the inorganic-inorganic coating powder, was prepared. When the total organic matter therein was quantitatively analyzed in Experimental Example 8, it was found that in the case of the composite powder coated with OMC at a concentration of 5 to 15%, more than 90% of the total organic matter was confirmed, and in the case of the composite powder coated with BMDBM at a concentration of 2 to 10%, 90% of the total organic matter applied to the coating was confirmed, thereby confirming that most of the inorganic matter applied to the OMC and BMDBM coated powders was coated.

Further, the average particle size of the organic-inorganic composite powders of the present invention was measured through Experimental Example 9, and as a result, a uniform range of average particle size was observed regardless of the coating concentrations of the UV inorganic absorbent, thereby confirming that all sizes were suitable which can be applied to cosmetics.

Furthermore, the UV blocking index of the organic-inorganic composite powders of the present invention was analyzed through Experimental Example 10, and as a result, they also showed a high level of SPF and UVA PF in proportion to the concentration of UV inorganic absorbent applied to coating. When coating the same type of organic absorbent, it showed a much higher UV blocking ability than ZnO, the base material, when the base material is ZnO, and when the same concentration of the organic absorbent was used, it showed a higher UV blocking ability when BMDBM was coated compared to when OMC was coated.

In addition, the functional ingredients of the organic-inorganic composite powders of the present invention were analyzed through Experimental Example 11, and as a result, in the case of the OMC-coated powder, it was quantified in proportion to the coating concentration from 5 to 15%, and the BMDBM coated powder was quantified in proportion to the coating concentration from 2 to 10%. Accordingly, it was found that there exists a critical concentration of OMC and BMDBM at which the powder is coated onto the base material.

From the results of Experiment Examples 10 to 11, it was found that the optimum coating concentration of the UV organic absorbent in Example 3 was preferably 5 to 15% by weight in the case of ethylhexyl methoxycinnamate and 2 to 10% by weight in the case of butylmethoxydibenzoylmethane.

From the above experiments, it has been found that the organic-inorganic composite powders of the present invention can be used as a powder cosmetic composition having enhanced ultraviolet protection function, and accordingly, multicut loose powder and multicut two way cake powder cosmetics containing the organic-inorganic composite powder of the present invention as an active ingredient were prepared (Example 4). In order to determine the ultraviolet protection function and skin harmfulness of the cosmetics, Experimental Example 13 was carried out. As a result, when the ultraviolet protection indexes (SPF, UVA PF) of multicut loose powder and multicut two way cake which applied the OMC and BMDBM coated powers of the present invention were examined by clinical and nonclinical methods, the SPF 50 and UVA PF of the two products were measured to be 8 or higher. Moreover, as a result of a skin irritation test by clinical evaluation, it was confirmed that the two products did not cause any skin irritation.

**Example 1. Preparation of UV blocking functional inorganic-inorganic coating powder of the present invention**

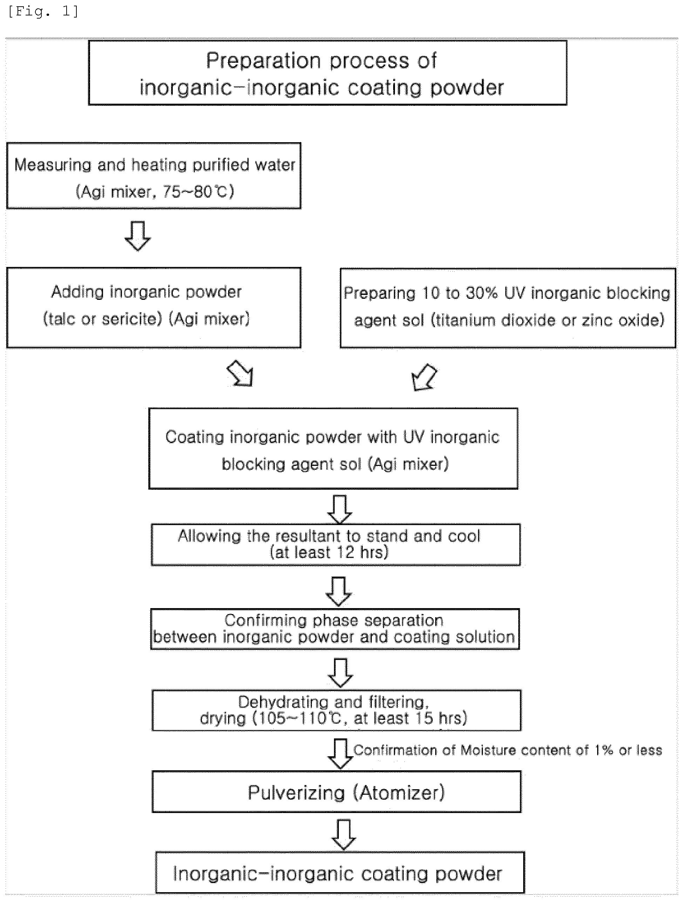

The preparation process of the UV blocking inorganic-inorganic coating powder of the present invention is shown in FIG. 1.

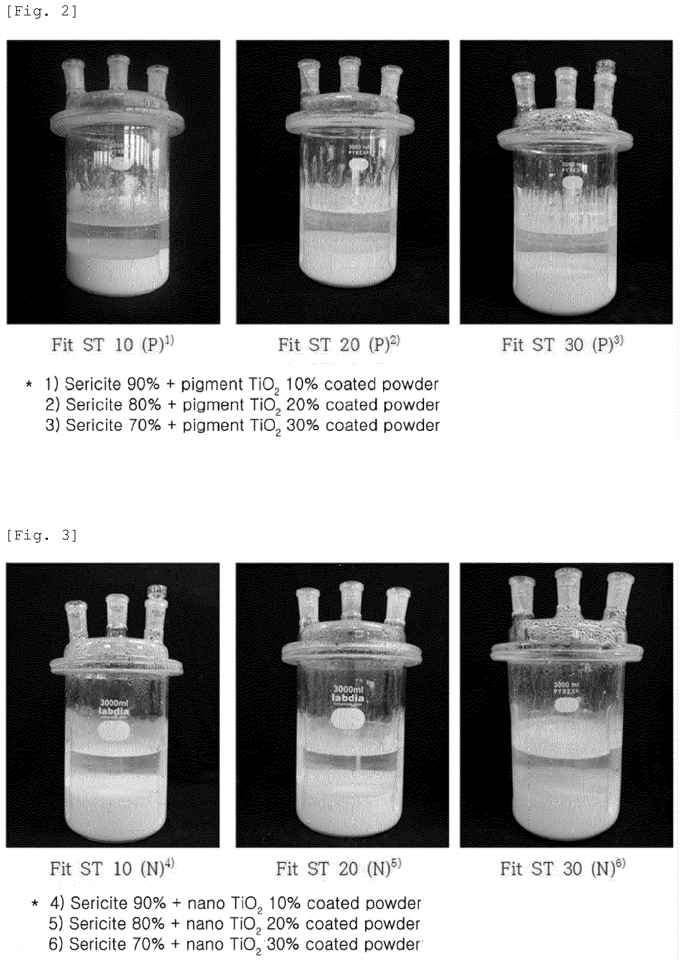

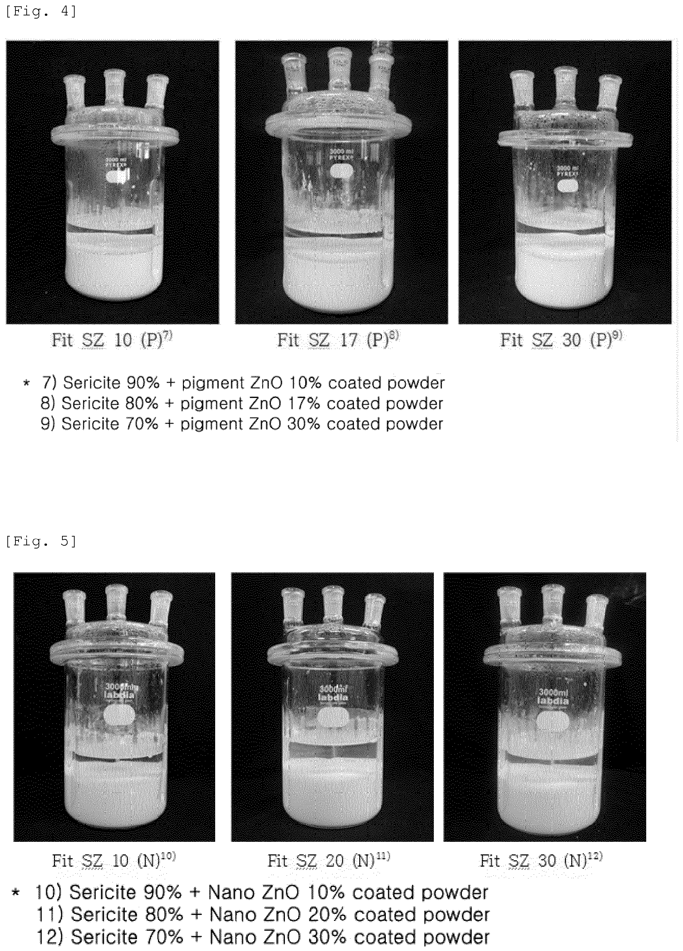

Specifically, after heating purified water at a temperature of 75°C to 80°C, 70 to 90% by weight of sericite was added to the purified water as a coating base material, and the mixture was stirred, mixed and dispersed. Separately, 10 to 30% titanium dioxide or zinc oxide, which are UV inorganic blocking agents, was added and dispersed in purified water with a bead mill to prepare a titanium dioxide sol or a zinc oxide sol. Then, the 10 to 30% titanium dioxide sol or zinc oxide sol was added to the coating base dispersion, followed by mixing and stirring the mixture for 25 to 30 minutes and then subjecting the same to aqueous coating. Thereafter, the coating was confirmed by the phase separation between the coating powder and purified water and the transparency of the supernatant by allowing the coating solution to stand for at least 12 hours and cool at room temperature (FIGS. 2 to 5). The coating powder was recovered from the phase-separated coating solution and dried at 105 to 110°C for 15 hours or more to make the water content to 1% or less. Then, the dried coating powder was pulverized using an atomizer to make uniform particles, thereby preparing an ultraviolet protection functional inorganic-inorganic coating powder of the present invention.

The inorganic-inorganic coating powder was prepared as shown in Table 1 according to the mixing ratio of the inorganic powder and the inorganic blocking agent, the type and size of the inorganic blocking agent. The size of the inorganic blocking agent used in the present invention includes a pigment unit having a particle size of 0.25 to 0.30 $\mu$m and a nano unit having a particle size of 30 to 50 nm. The indication of the inorganic-inorganic coating powder of Table 1 is as follows: in the case of Fit ST 10 (P), Fit is an inorganic-inorganic coating powder, S is an abbreviation for sericite, a type of inorganic powder, T is an abbreviation for $TiO_2$, a type of UV blocking agent, and (P) represents pigment, which is a formulation of the UV blocking agent. Hereinafter, the inorganic-inorganic coating powder was described by the description method above.

[Table 1]

| Inorganic-inorganic coating powders | Inorganic powder (wt%) | | UV inorganic blocking agent (wt%) | | | |
|---|---|---|---|---|---|---|
| | Talc | Sericite | Pigment $TiO_2$ | Nano $TiO_2$ | Pigment ZnO | Nano ZnO |
| Fit ST 10(P) | - | 90 | 10 | - | - | - |

(continued)

| Inorganic-inorganic coating powders | Inorganic powder (wt%) | | UV inorganic blocking agent (wt%) | | | |
|---|---|---|---|---|---|---|
| | Talc | Sericite | Pigment TiO$_2$ | Nano TiO$_2$ | Pigment ZnO | Nano ZnO |
| Fit ST 20(P) | - | 80 | 20 | - | - | - |
| Fit ST 30(P) | - | 70 | 30 | - | - | - |
| Fit ST 10(N) | - | 90 | - | 10 | - | - |
| Fit ST 20(N) | - | 80 | - | 20 | - | - |
| Fit ST 30(N) | - | 70 | - | 30 | - | - |
| Fit SZ 10(P) | - | 90 | - | - | 10 | - |
| Fit SZ 20(P) | - | 80 | - | - | 20 | - |
| Fit SZ 30(P) | - | 70 | - | - | 30 | - |
| Fit SZ 10(N) | - | 90 | - | - | - | 10 |
| Fit SZ 20(N) | - | 80 | - | - | - | 20 |
| Fit SZ 30(N) | - | 70 | - | - | - | 30 |

**Experimental Example 1. Observation of phase-separation of inorganic-inorganic coating powders**

In order to confirm the coating of the inorganic-inorganic coating powders of Table 1 prepared according to the preparation process of Example 1, the phase separation of the coating solution was observed.

As shown in FIGS. 2 to 5, the phase separation of the coating solution was carried out such that all the twelve inorganic-inorganic coating powders shown in Table 1 were clearly phase-separated from the purified water, and accordingly it was confirmed through the preparation process of the Example that the UV inorganic blocking agent was properly coated onto the inorganic powders.

**Experimental Example 2. Evaluation of transparency of filtrate after preparing inorganic-inorganic coating powders**

After filtering the coating solution prepared in Example 1, the transparency of the filtrate was confirmed. If there existed powder that was not coated on the coating solution, the transparency of the powder would be poorer than that of the purified water, and thus, it could be confirmed that whether the coating was carried out properly.

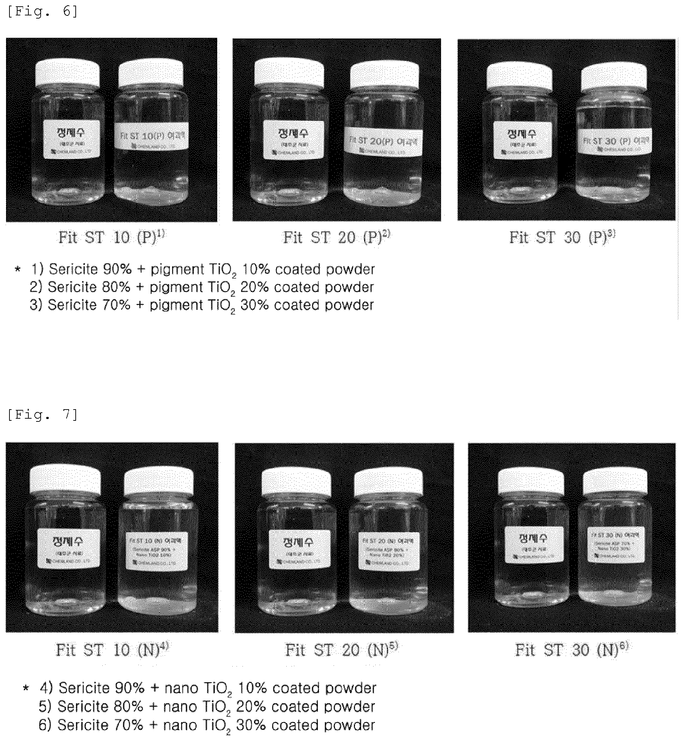

As shown in FIG. 6, the transparency of the filtrate was not significantly different from that of purified water for all of the twelve inorganic-inorganic coated powder filtrates of Table 1, and from this, it was confirmed that the UV blocking agent was properly coated on the coating powder through the preparation process of the Example.

**Experimental Example 3. FE-SEM of inorganic-inorganic coating powders**

FE-SEM refers to a field emission scanning electron microscope, which uses a field emission electron gun to confirm magnified images of materials in the size of 0.5 to 2.0 nm with a resolution power of up to 1 million times and provides a higher quality image with a high-resolution power.

The inorganic-inorganic coating powders of the present invention were observed at a maximum magnification of 10,000 times at 15 kV to confirm the surface of the coating powders. The equipment analysis was carried out by the Korea Ceramic Technology Institute.

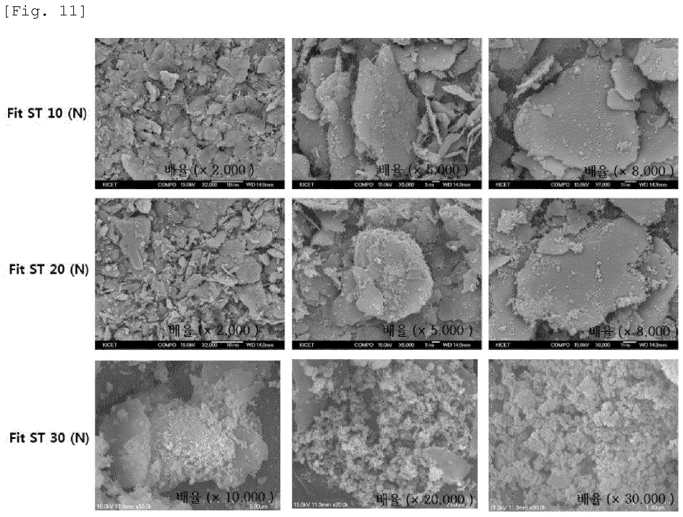

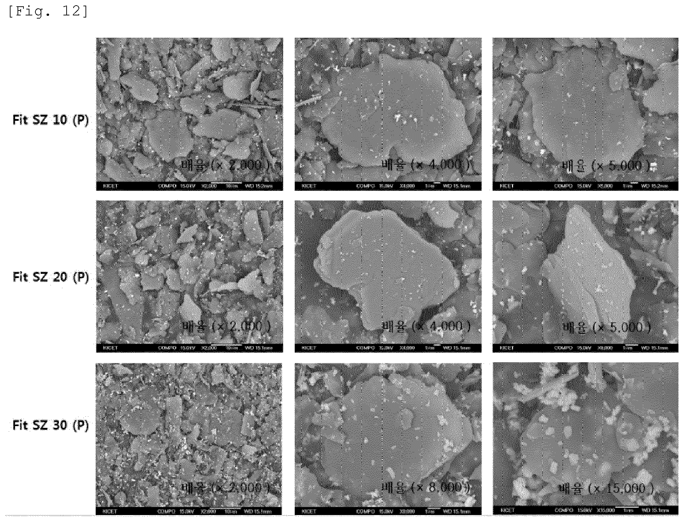

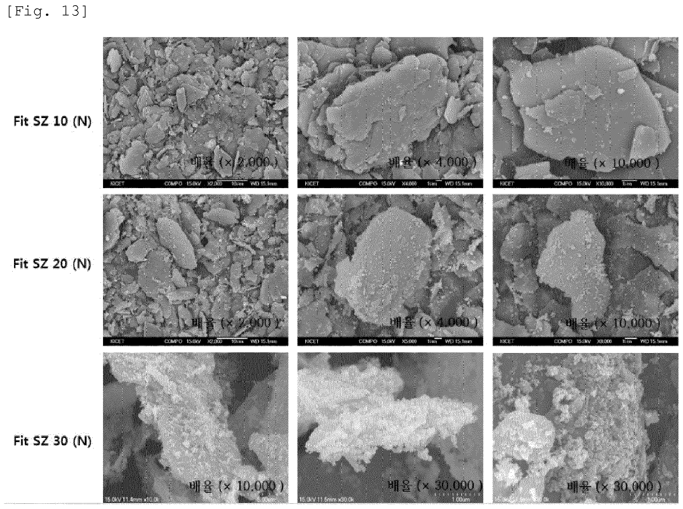

As shown in FIGS. 11 to 13, when confirming the surface of the twelve inorganic-inorganic coating powders shown in Table 1 by FE-SEM, it was confirmed that white titanium dioxide or zinc oxide was evenly coated on the sericite with grey background.

**Experimental Example 4. EDS of inorganic-inorganic coating powders**

Based on the FE-SEM results of Experimental Example 3, the elements of each powder surface were analyzed to confirm whether titanium dioxide or zinc oxide used as a coating agent was properly present in the coating powder by using EDS. EDS (Energy Dispersive X-ray Spectroscopy) is a kind of element analyzer and is a device that quantifies its content ratio by decomposing the element unit when an ingredient present in a specific substance exists as a metal salt.

[Table 2]

| Analysis result of EDS (unit: %) | | | | | | | | | | |
|---|---|---|---|---|---|---|---|---|---|---|
| Inorganic-inorganic coating powder | O | Na | Mg | Al | Si | K | Ti | Fe | Zn | Total |
| Fit ST 10(P) | 59.45 | 0.35 | 0.75 | 12.76 | 17.89 | 6.81 | 1.69 | 1.67 | - | 100 |
| Fit ST 20(P) | 63.51 | 0.61 | 0.96 | 11.92 | 15.23 | 5.12 | 2.71 | 0.70 | - | 100 |
| Fit ST 30(P) | 59.47 | 0.65 | 1.03 | 11.51 | 15.35 | 5.68 | 6.26 | 0.83 | - | 100 |
| Fit ST 10(N) | 63.78 | 0.68 | 0.56 | 12.72 | 14.92 | 4.96 | 2.44 | 0.96 | - | 100 |
| Fit ST 20(N) | 61.36 | 0.73 | 1.05 | 12.08 | 14.94 | 5.12 | 5.02 | 0.66 | - | 100 |
| Fit ST 30(N) | 60.55 | 0.54 | 1.12 | 11.45 | 15.13 | 5.18 | 6.48 | 0.79 | - | 100 |
| Fit SZ 10(P) | 60.46 | 0.87 | 0.71 | 13.26 | 16.29 | 5.55 | - | 1.05 | 2.97 | 100 |
| Fit SZ 20(P) | 61.47 | - | - | 11.75 | 12.71 | 4.26 | - | 0.61 | 9.52 | 100 |
| Fit SZ 30(P) | 53.78 | - | 0.53 | 10.13 | 11.32 | 3.46 | - | 0.48 | 20.97 | 100 |
| Fit SZ 10(N) | 52.79 | - | 0.93 | 13.31 | 18.26 | 8.36 | - | 1.56 | 4.81 | 100 |
| Fit SZ 20(N) | 54.20 | - | 0.25 | 11.58 | 12.15 | 4.30 | - | 0.46 | 17.20 | 100 |
| Fit SZ 30(N) | 52.66 | - | 0.3 | 11.1 | 12.75 | 4.13 | - | 0.61 | 18.61 | |

As shown in Table 1, given that the typical composition of sericite exists in the form of a metal salt such as $SiO_2$ (70.12%), $Al_2O_3$ (17.97%), $Fe_2O_3$ (0.71%), CaO (0.27%), MgO (1.36%), $K_2O$ (6.07%), $Na_2O$ (0.14%) and the like, the elements constituting sericite and the elements of titanium dioxide ($TiO_2$) or zinc oxide (ZnO) used as the coating material were all identified in the twelve types of inorganic-inorganic coating powders shown in Table 1.

The average composition ratio of Ti- in 10 to 30% titanium dioxide used as the coating material was about 1.69 to 6.48% and the average composition ratio of 10 to 30% Zn-was about 2.97 to 20.97%. In addition, it can be found that considering that metals such as Mg-, Fe-, Na- and the like are present in sericite in a trace amount, the detection thereof was not uniform.

As a result, it was quantitatively confirmed in the elemental unit that titanium oxide or zinc oxide, which is a coating agent, and sericite, which is a coating base, were properly coated on the inorganic-inorganic coating powder of the present invention prepared by the preparation process of Example 1.

**Experimental Example 5. Analysis of purity of inorganic-inorganic coating powders**

In this experiment, the contents of titanium dioxide and zinc oxide in the titanium dioxide and zinc oxide coated powders coated with different sizes and concentrations, which were analyzed so far, were analyzed. This is to confirm whether the inorganic matter which was surface-treated on the coating base material was properly coated to a desired concentration. This experiment was carried out by Korea Institute of Construction & Living Environment Research Institute, an authorized analysis institute designated by the Food and Drug Administration.

[Table 3]

| Inorganic-inorganic coating powders | Target for analysis | Content (wt%) |
|---|---|---|
| Fit ST 10(P) | Titanium dioxide | 8.19 |
| Fit ST 20(P) | Titanium dioxide | 20.31 |
| Fit ST 30(P) | Titanium dioxide | 27.80 |
| Fit ST 10(N) | Titanium dioxide | 10.71 |
| Fit ST 20(N) | Titanium dioxide | 19.85 |
| Fit ST 30(N) | Titanium dioxide | 28.88 |

(continued)

| Inorganic-inorganic coating powders | Target for analysis | Content (wt%) |
|---|---|---|
| Fit SZ 10(P) | Zinc Oxide | 9.57 |
| Fit SZ 20(P) | Zinc Oxide | 15.31 |
| Fit SZ 30(P) | Zinc Oxide | 28.91 |
| Fit SZ 10(N) | Zinc Oxide | 9.37 |
| Fit SZ 20(N) | Zinc Oxide | 18.28 |
| Fit SZ 30(N) | Zinc Oxide | 30.9 |

As shown in Table 3, the coating concentration of the UV blocking agent coated on the inorganic powders was found to be about 8.19 to 28.88% for 10 to 30% titanium dioxide and about 9.37 to 30.9% for 10 to 30% zinc oxide, and thus it was confirmed the inorganic powder was well coated with titanium dioxide or zinc oxide in the inorganic-inorganic coating powders.

**Experimental Example 6. UV blocking index of inorganic-inorganic coating powders**

The UV blocking index was measured for the twelve composite powders coated with titanium dioxide and zinc oxide to examine the difference in ultraviolet protection ability between each powder. This is to confirm the UV blocking index of composite powders coated with titanium dioxide and zinc oxide with different sizes and to select a base material to be used for OMC and BMDBM coating. The SPF, UVA-PF and critical wavelength (C.W) between the coating powers coated with different sizes and concentrations were confirmed through a SPA 290S model, which is a UV measuring device.

[Table 4]

| Inorganic-inorganic coating powders | SPF | UVA-PE |
|---|---|---|
| Fit ST 10(P) | 6.3 | 5.81 |
| Fit ST 20(P) | 14.38 | 13.14 |
| Fit ST 30(P) | 20.74 | 19.36 |
| Fit ST 10(N) | 18.22 | 10.37 |
| Fit ST 20(N) | 24.48 | 16.38 |
| Fit ST 30(N) | 39.27 | 24.04 |
| Fit SZ 10(P) | 3.55 | 3.35 |
| Fit SZ 20(P) | 4.09 | 3.95 |
| Fit SZ 30(P) | 8.12 | 7.83 |
| Fit SZ 10(N) | 4.96 | 4.19 |
| Fit SZ 20(N) | 7.35 | 6.13 |
| Fit SZ 30(N) | 9.64 | 8.34 |

As shown in Table 4, the $TiO_2$ coated powder was superior in protection ability in the ultraviolet A and B regions compared to the ZnO coated powder. In addition, for all coating powders, the UV blocking index also increased in proportion to the increase in the concentration of the UV blocking agent. However, these values were slightly different according to the particle size of the UV blocking agent, and the UV blocking agent of nano grade was higher than that of pigment Grade. It is generally known that $TiO_2$ and ZnO have a protection ability against ultraviolet-B and ultraviolet-A regions, respectively, and those having an average particle size of 100 nm (0.1 $\mu$m) or less have excellent protection ability against ultraviolet rays. $TiO_2$ and ZnO having a size of 100 nm or higher also have a certain level of ultraviolet protection ability, but they are rarely used because the particle size thereof is about 10 times larger than that of the nano-sized powder, and thus it causes a white cloudy phenomenon in skin when applied to basic and color cosmetics.

Therefore, by summarizing the above results, a composite powder coated with 30% of nano-sized UV inorganic blocking agent was selected as a base material for coating the OMC and BMDBM which are UV inorganic absorbents.

**Example 2. Preparation of UV blocking functional organic-inorganic composite powder of the present invention**

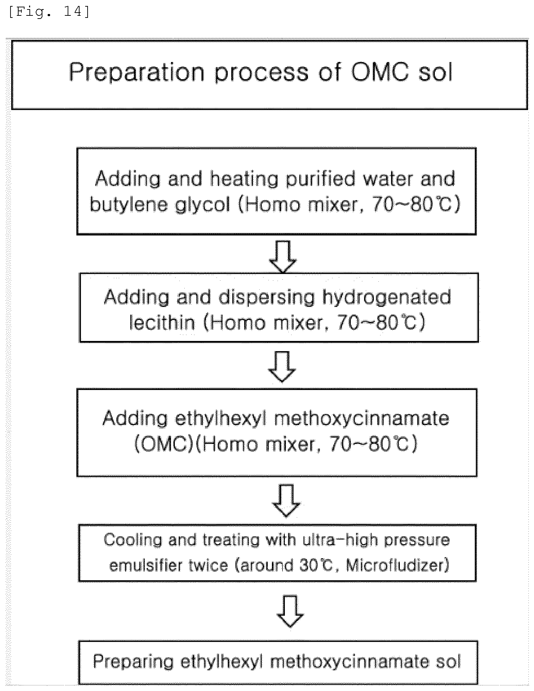

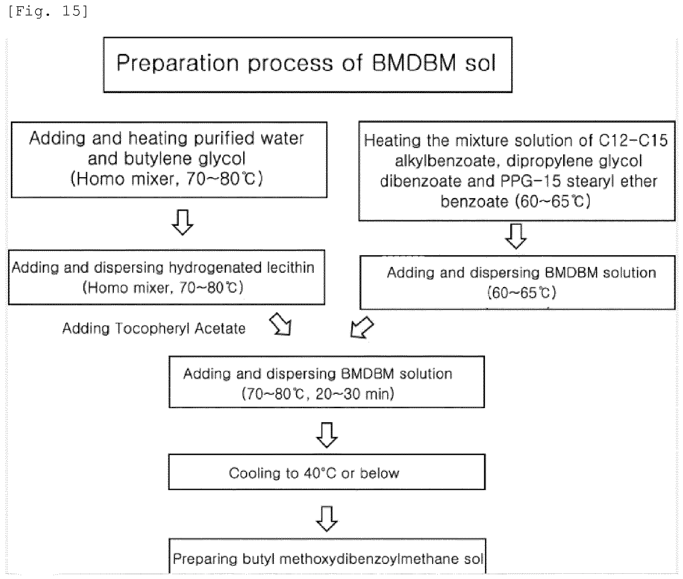

The present invention relates to a process for coating an ultraviolet organic absorbent encapsulated into a liposome on the previously coated inorganic-inorganic composite powder. The preparation process of a UV inorganic absorbent sol required for carrying out a coating depends on the kind of the ultraviolet organic absorbents. The preparation process for ethylhexyl methoxycinnamate (OMC) is as shown in FIG. 14 and butyl methoxydibenzoylmethane (BMDBM) as shown in FIG. 15.

Specifically, the preparation process of the OMC sol was carried out by first heating 500 to 900% by weight of purified water to 70 to 80°C, adding 1 to 5% by weight of butylene glycol and 0.1 to 1% by weight of tocopheryl acetate thereto and stirring, mixing and dispersing the mixture, then adding and dispersing 1 to 10% by weight of hydrogenated lecithin at the same temperature range. Thereafter, 5 to 20% by weight of ethylhexyl methoxycinnamate, which is an UV inorganic absorbent, was added and dispersed, followed by subjecting it to liposomation. In the step above, when ethylhexyl methoxycinnamate was added, the mixture was cooled to about 30°C, treated twice with an ultrahigh-pressure emulsifier, and then cooled to about 40°C to prepare an OMC sol for coating the inorganic-inorganic composite powder (FIG. 14).

On the other hand, the preparation process of BMDBM was carried out by first heating 500 to 900% by weight of purified water to 70 to 80°C, adding 1 to 5 % by weight of butylene glycol and 0.1 to 1.0% by weight of tocopheryl acetate thereto and stirring, mixing and dispersing the mixture, then adding and dispersing 1 to 10% by weight of hydrogenated lecithin at the same temperature range to prepare a liposome mixture solution. Separately, 2 to 10% by weight of BMDBM was dissolved in a mixture solution containing equivalent amount of 3 to 15% by weight of $C_{12}$-$C_{15}$ alkyl benzoate, dipropylene glycol dibenzoate and PPG-15 stearyl ether benzoate as being heated to 60 to 65°C to prepare a BMDBM solution. Thereafter, 2 to 10% by weight of the BMDBM solution prepared above was added and dispersed in the prepared liposome mixture, and the mixture was cooled to about 40°C at room temperature to prepare a BMDBM sol for coating the inorganic-inorganic composite powder (FIG. 15).

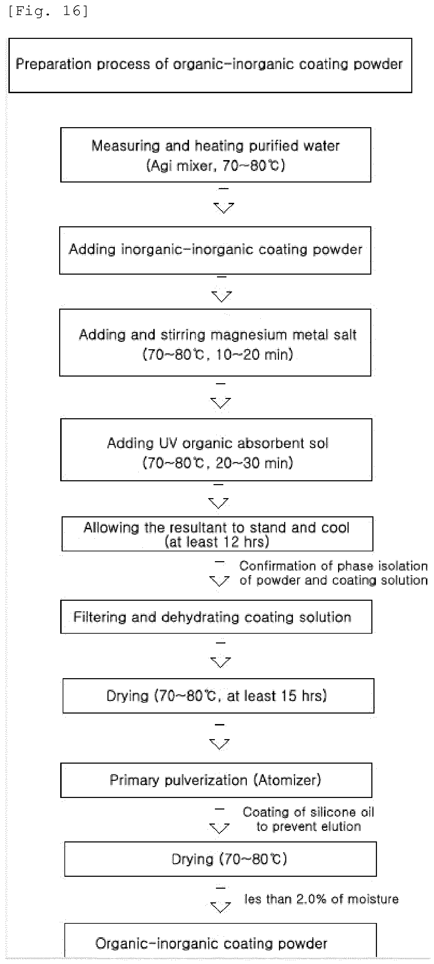

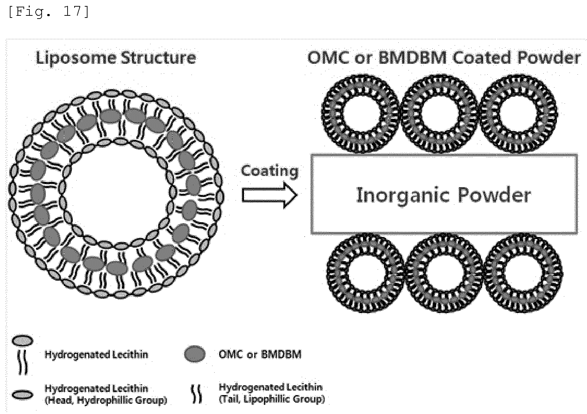

The preparation process of the UV blocking functional organic-inorganic composite powder of the present invention is shown in FIG. 16. Specifically, 70 to 95% by weight of Fit TT 30 (N) or Fit ST 30 (N) coated powder of Example 1, as a coating base material, and 0.1 to 5% by weight of magnesium metal salt were added to 500 to 900% by weight of purified water heated to 70 to 80°C based on the weight of purified water, and the mixture was stirred for 20 to 30 minutes. Then, the OMC sol or BMDBM sol prepared as shown in FIGS. 14 to 15 was added as a coating agent, stirred and subjected to aqueous coating onto the inorganic-inorganic coating powder. Thereafter, the phase separation and transparency of the supernatant was confirmed by allowing the coating solution to stand for at least 12 hours and cool, and the coating solution was filtered, dehydrated, and then dried at 70-80°C for at least 15 hours to adjust the water content to 2% or less. The dried product obtained in the step above was first pulverized and used as a coating base, then it was subjected to dry coating with triethoxycarrylylsilane (OTS), which is a silicone oil, at a concentration of 3% by weight. Then, the resultant was dried again to prepare UV blocking functional organic-inorganic composite powders of the present invention (FIG. 17)

The organic-inorganic coating powders were prepared as shown in Table 5 according to the mixing ratio of the inorganic powder, the inorganic blocking agent and the organic absorbent, the type of the inorganic blocking agent, and the type of the organic absorbent.

As for the indication of the organic-inorganic coating powders of Table 5, Artisto represents OMC coating and Vasto represents BMDBM, for example, in the case of Artisto TZ05, Artisto represents OMC coating, T is an abbreviation for Talc, an inorganic powder type, Z is an abbreviation of nano ZnO, an UV inorganic blocking agent. Hereinafter, the organic-inorganic coating powders were described by the description method above.

[Table 5]

| (wt%) | | | | | | |
|---|---|---|---|---|---|---|
| Organic-inorganic | Inorganic-inorganic coating | | | | UV organic | |
| coating powder | powder | | | | absorbent sol | |
| | Fit TT 30 (N) | Fit ST 30 (N) | Fit TZ 30 (N) | Fit SZ 30 (N) | OMC | BMDBM |
| Artisto TT05 | 78-94 | - | - | - | 5 | - |
| Artisto TT10 | 73-89 | - | - | - | 10 | - |

(continued)

| (wt%) | | | | | | |
|---|---|---|---|---|---|---|
| Organic-inorganic | Inorganic-inorganic coating | | | | UV organic | |
| coating powder | powder | | | | absorbent sol | |
| | Fit TT 30 (N) | Fit ST 30 (N) | Fit TZ 30 (N) | Fit SZ 30 (N) | OMC | BMDBM |
| Artisto TT15 | 68-84 | - | - | - | 15 | - |
| Artisto TT20 | 63-79 | - | - | - | 20 | - |
| Artisto ST05 | - | 78-94 | - | - | 5 | - |
| Artisto ST10 | - | 73-89 | - | - | 10 | - |
| Artisto ST15 | - | 68-84 | - | | 15 | |
| Artisto ST20 | - | 63-79 | - | - | 20 | - |
| Artisto TZ05 | - | - | 78-94 | - | 5 | - |
| Artisto TZ10 | - | - | 73-89 | - | 10 | - |
| Artisto TZ15 | | | 68-84 | - | 15 | - |
| Artisto TZ20 | - | - | 63-79 | - | 20 | - |
| Artisto SZ05 | - | - | - | 78-94 | 5 | - |
| Artisto SZ10 | - | - | - | 73-89 | 10 | - |
| Artisto SZ15 | - | - | - | 68-84 | 15 | - |
| Artisto SZ20 | - | - | - | 63-79 | 20 | - |
| Vasto TT02 | 66-94 | - | - | - | - | 2 |
| Vasto TT04 | 64-92 | - | - | - | - | 4 |
| Vasto TT06 | 62-90 | - | - | - | - | 6 |
| Vasto TT08 | 60-88 | - | - | - | - | 8 |
| Vasto TT10 | 58-86 | - | - | - | - | 10 |
| Vasto ST02 | - | 66-94 | - | - | - | 2 |
| Vasto ST04 | - | 64-92 | - | - | - | 4 |
| Vasto ST06 | - | 62-90 | - | - | - | 6 |
| Vasto ST08 | - | 60-88 | - | - | | 8 |
| Vasto ST10 | - | 58-86 | - | - | - | 10 |
| Vasto TZ02 | - | - | 66-94 | - | - | 2 |
| Vasto TZ04 | - | - | 64-92 | - | - | 4 |
| Vasto TZ06 | - | - | 62-90 | - | - | 6 |
| Vasto TZ08 | - | - | 60-88 | - | - | 8 |
| Vasto TZ10 | - | - | 58-86 | - | - | 10 |
| Vasto SZ02 | - | - | - | 66-94 | - | 2 |
| Vasto SZ04 | - | - | - | 64-92 | - | 4 |
| Vasto SZ06 | - | - | - | 62-90 | - | 6 |
| Vasto SZ08 | - | - | - | 60-88 | - | 8 |
| Vasto SZ10 | - | - | - | 58-86 | - | 10 |

**Experimental Example 7. Evaluation of phase separation of organic-inorganic coating powders and transparency of filtrates**

The phase separation of the coating solution and the transparency of the filtrate were observed to confirm the coating of the organic-inorganic coating powders of Table 5 prepared according to the preparation process of Example 2. To confirm the transparency of 36 coating powder filtrates of Example 2 more accurately, the absorbance was confirmed using a spectrophotometer (UV-spentrophotometer, 660 nm).

[Table 6]

| Organic-inorganic coating powder | Absorbance (660nm) |
|---|---|
| Artisto TT05 | 0.052 |
| Artisto TT10 | 0.065 |
| Artisto TT15 | 0.014 |
| Artisto TT20 | 0.048 |
| Artisto ST05 | 0.036 |
| Artisto ST10 | 0.020 |
| Artisto ST15 | 0.062 |
| Artisto ST20 | 0.046 |
| Artisto TZ05 | 0.034 |
| Artisto TZ10 | 0.036 |
| Artisto TZ15 | 0.020 |
| Artisto TZ20 | 0.051 |
| Artisto SZ05 | 0.028 |
| Artisto SZ10 | 0.029 |
| Artisto SZ15 | 0.053 |
| Artisto SZ20 | 0.037 |
| Vasto TT02 | 0.009 |
| Vasto TT04 | 0.032 |
| Vasto TT06 | 0.034 |
| Vasto TT08 | 0.051 |
| Vasto TT10 | 0.044 |
| Vasto ST02 | 0.025 |
| Vasto ST04 | 0.062 |
| Vasto ST06 | 0.046 |
| Vasto ST08 | 0.041 |
| Vasto ST10 | 0.059 |
| Vasto TZ02 | 0.021 |
| Vasto TZ04 | 0.017 |
| Vasto TZ06 | 0.018 |
| Vasto TZ08 | 0.026 |
| Vasto TZ10 | 0.010 |
| Vasto SZ02 | 0.004 |

(continued)

| Organic-inorganic coating powder | Absorbance (660nm) |
|---|---|
| Vasto SZ04 | 0.003 |
| Vasto SZ06 | 0.002 |
| Vasto SZ08 | 0.011 |
| Vasto SZ10 | 0.021 |

As shown in Table 6, the absorbance of 36 the organic-inorganic coating powders of Example 2 increased as the coating concentration increased, but the range was 0.002 to 0.065, which was similar to the absorbance (0) of purified water. In addition, all 36 coating powders showed a good phase separation between the coating solution and the coating powder. From this, it was confirmed that the UV absorbent was properly coated on the inorganic-inorganic coating powder through the preparation process of Example 2.

**Experimental Example 8. Quantitative Analysis of organic-inorganic coating powders (TG-DSC)**

All organic matters contained in the organic-inorganic coating powders of the present invention were quantitatively analyzed to confirm whether the organic matter applied to the present coating was properly coated on the base material. The quantitative analysis of organic matter was carried out by the Korea Ceramic Research Institute via TG-DSC method, and as an analytical equipment, a differential scanning calorimeter (Model: STA409PC, Luxx) was used by heating it to 800°C at a rate of 10°C per minute to measure an increase or decrease, and endothermic or exothermic phenomenon of the sample weights (Initiation temperature: room temperature, Relative humidity: 50.1±1%, Heating rate per minute: 5°C/min, $N_2$ Gas: 30 ml/min). As a control group, the inorganic-inorganic coating powder before coating of the UV absorbent of each organic-inorganic coating powder was used.

[Table 7]

| 1 | Total concentrati on of organic matter applied for preparation (%) | TG-DSC result of experimental group (%) | TG-DSC result of control group (%) | Actual concentrati on coated on powder 1) | Coating yield 2) |
|---|---|---|---|---|---|
| Artisto TT05 | 6.625 | 10.37 | 2.13 | 8.24 | 124 |
| Artisto ST05 | | 9.60 | 3.68 | 5.92 | 89 |
| Artisto TT10 | 13.25 | 14.83 | 2.13 | 12.7 | 96 |
| Artisto ST10 | | 15.15 | 3.68 | 11.47 | 87 |
| Artisto TT15 | 19.875 | 26.93 | 2.13 | 24.8 | 125 |
| Artisto ST15 | | 22.11 | 3.68 | 18.43 | 93 |
| Artisto TT20 | 26.5 | 18.32 | 2.13 | 16.19 | 61 |
| Artisto ST20 | | 21.09 | 3.68 | 17.41 | 66 |
| Vasto TT02 | 9.5 | 11.51 | 2.13 | 9.38 | 98.7 |
| Vasto ST02 | | 11.91 | 3.68 | 8.23 | 86.6 |

(continued)

| 1 | Total concentrati on of organic matter applied for preparation (%) | TG-DSC result of experimental group (%) | TG-DSC result of control group (%) | Actual concentrati on coated on powder 1) | Coating yield 2) |
|---|---|---|---|---|---|
| Vasto TT04 | 14.6 | 16.38 | 2.13 | 14.25 | 98.3 |
| Vasto ST04 | | 16.60 | 3.68 | 12.92 | 89.1 |
| Vasto TT06 | 19.5 | 18.17 | 2.13 | 16.04 | 82.3 |
| Vasto ST06 | | 21.19 | 3.68 | 17.51 | 89.8 |
| Vasto TT08 | 24.5 | 24.47 | 2.13 | 22.34 | 91.2 |
| Vasto ST08 | | 25.18 | 3.68 | 21.50 | 87.8 |
| Vasto TT10 | 29.5 | 26.96 | 2.13 | 24.83 | 84.2 |
| Vasto ST10 | | 30.63 | 3.68 | 26.95 | 91.4 |
| 1 Actual concentration coated on powder= [TG-DCS measured value (%) - corrected density (%)], 2) coating yield (%) = (Actual coating concentration / total concentration of organic matter) x 100 | | | | | |

As shown in Table 7, the coating yield of OMC was 60 to 65% when the coating concentration was 20%, which was ineffective, and it was preferable that the coating concentration was 15% or less. In addition, the coating yield of BMDBM was the best at 2% and 4%, and a yield of 90% or higher was obtained at 8%.

**Experimental Example 9. Analysis of particle size of organic-inorganic coating powders**

The average particle size (D50) of the organic-inorganic coating powders of the present invention was measured. Generally, the ingredients (including the coating powder) used in powder formulations such as two way cake and loose powder are used by surface treating inorganic powders having an average particle size of 30 $\mu$m or less with various coating agents (for example, silicone oil for water repellency). These coating powders can provide spreadability, adhesion and moisturizing power, etc. in powder products in addition to water repellency.

In the powder cosmetics, inorganic powder having an average particle size of about 20 $\mu$m or less and 30 $\mu$m or less is mainly used for loose powder. When the loose powder is used as a formulation in which a constant pressure is not applied, a puff and a brush for loose powder are used. A smaller amount of powder is smeared into the puff and brush used in the loose powder than for two way cake, and such small amount of powder must be evenly applied to the skin. If the particle size of the coating powder applied to lose powder is small, it may be difficult to be uniformly applied to the face. When such small size-powder is coated, it may improve spreadability and applicability to a certain level, but it may be difficult to change the intrinsic properties of the powder only by coating since the feeling of use of the ingredients may remain after coating. The inorganic powder (including the coating powder) used in the loose powder is a powder having a relatively large particle size having an average particle size of about 20 $\mu$m or less or 30 $\mu$m or less, and the deficient properties of the ingredients may be improved by surface treatment (coating) with various coating agents.

Therefore, whether the organic-inorganic coating powders of the present invention were applicable to powder cosmetics was confirmed by measuring the average particle size thereof. The particle size analysis was performed using a particle size analyzer of Microtrac (USA, model: S3500), and the powder was dispersed in IPA at a concentration of 0.3% (w/w) and ultrasonicated to measure the average particle size. The average particle size of the coating powder was analyzed and at the same time, the particle size distribution was confirmed through the particle size distribution graph.

[Table 8]

| Organic-Inorganic Coating Powder | Average Particle Size (μm) |
|---|---|
| Artisto TT05 | 17.68 |
| Artisto TT10 | 19.90 |
| Artisto TT15 | 24.01 |
| Artisto TT20 | 21.61 |
| Artisto ST05 | 8.76 |
| Artisto ST10 | 7.74 |
| Artisto ST15 | 9.07 |
| Artisto ST20 | 12.74 |
| Artisto TZ05 | 19.37 |
| Artisto TZ10 | 23.41 |
| Artisto TZ15 | 21.24 |
| Artisto TZ20 | 22.64 |
| Artisto SZ05 | 9.37 |
| Artisto SZ10 | 9.64 |
| Artisto SZ15 | 9.95 |
| Artisto SZ20 | 9.93 |
| Vasto TT02 | 18.89 |
| Vasto TT04 | 18.44 |
| Vasto TT06 | 18.77 |
| Vasto TT08 | 19.12 |
| Vasto TT10 | 18.72 |
| Vasto ST02 | 8.66 |
| Vasto TT04 | 9.23 |
| Vasto ST06 | 9.35 |
| Vasto ST08 | 8.72 |
| Vasto ST10 | 8.54 |
| Vasto TZ02 | 20.31 |
| Vasto TT04 | 19.14 |
| Vasto TZ06 | 20.01 |
| Vasto TZ08 | 19.92 |
| Vasto TZ10 | 19.34 |
| Vasto SZ02 | 9.31 |
| Vasto TT04 | 10.63 |
| Vasto SZ06 | 10.58 |
| Vasto SZ08 | 10.85 |
| Vasto SZ10 | 9.59 |

As shown in Table 8, for the average particle size of the 36 organic-inorganic coating powders of Example 2, the concentration of the organic matter applied in the coating tended to increase as the coating concentration of the organic absorbent increased, but it had no influence on the average particle size of the coating powders, and all 36 coating powders were applicable as powder cosmetics as the particle sizes thereof were all 30 μm or less.

**Experimental Example 10. UV blocking index of organic-inorganic coating powders**

The UV blocking index of the organic-inorganic coating powders of the present invention was measured. This is to select the coating powders to be applied to two way cake or loose powder mentioned above, and whether there is a difference in the UV blocking ability between each coating powder was also confirmed. This analysis used the same analytical equipment (equipment name: SPF 290S) as in the measurement of ultraviolet protection indexes of the $TiO_2$ and ZnO coated powders

[Table 9]

| Organic-Inorganic Coating Powder | UV blocking Index | |
|---|---|---|
| | SPF | UVA PF |
| Artisto TT05 | 38.89 | 18.19 |
| Artisto TT10 | 45.46 | 18.22 |
| Artisto TT15 | 76.72 | 28.21 |
| Artisto TT20 | 63.85 | 22.37 |
| Artisto ST05 | 77.95 | 36.45 |
| Artisto ST10 | 99.35 | 39.99 |
| Artisto ST15 | 179.38 | 76.85 |
| Artisto ST20 | 159.57 | 66.18 |
| Artisto TZ05 | 9.87 | 5.49 |
| Artisto TZ10 | 20.84 | 8.46 |
| Artisto TZ15 | 21.83 | 7.95 |
| Artisto TZ20 | 38.39 | 12.51 |
| Artisto SZ05 | 18.81 | 8.83 |
| Artisto SZ10 | 29.42 | 11.98 |
| Artisto SZ15 | 37.86 | 13.55 |
| Artisto SZ20 | 57.42 | 19.74 |
| Vasto TT02 | 54.29 | 32.89 |
| Vasto TT04 | 73.78 | 65.42 |
| Vasto TT06 | 85.00 | 100.83 |
| Vasto TT08 | 112.09 | 118.88 |
| Vasto TT10 | 114.85 | 123.48 |
| Vasto ST02 | 62.51 | 35.4 |
| Vasto ST04 | 112.43 | 78.92 |
| Vasto ST06 | 157.57 | 125.75 |
| Vasto ST08 | 188.2 | 173.92 |
| Vasto ST10 | 177.24 | 180.79 |
| Vasto TZ02 | 9.5 | 8.8 |
| Vasto TZ04 | 10.13 | 9.62 |
| Vasto TZ06 | 13.97 | 14.14 |

(continued)

| Organic-Inorganic Coating Powder | UV blocking Index | |
| --- | --- | --- |
| | SPF | UVA PF |
| Vasto TZ08 | 14.24 | 15.34 |
| Vasto TZ10 | 15.54 | 17.99 |
| Vasto SZ02 | 16.87 | 15.32 |
| Vasto SZ04 | 22.01 | 21.38 |
| Vasto SZ06 | 22.16 | 25.1 |
| Vasto SZ08 | 26.23 | 31.74 |
| Vasto SZ10 | 25.53 | 32.56 |

As shown in Table 9, the UV blocking index of the 36 organic-inorganic coating powders of Example 2 increased in proportion to the OMC concentration when 5 to 15% OMC was coated on the $TiO_2$ base material, but it rather decreased when 20% OMC was coated. Accordingly, it was confirmed that the optimum coating concentration of OMC for $TiO_2$ base material was 15%. In addition, when the coating concentration of OMC was the same, the UV blocking index was 2 times higher or more when $TiO_2$ was the inorganic blocking agent used as a base material than when ZnO was used. There was a significantly large difference in the ultraviolet protection ability depending on the types of ultraviolet blocking agents used as base materials.

Further, in general, all of the composite powders coated with two different kinds of inorganic blocking agents showed a significantly higher ultraviolet protection ability when $TiO_2$ was used as a base material. When the same amount of the two organic absorbents were used, it showed a much more superior ultraviolet protection ability when BMDBM was coated rather than OMC. When comparing the ultraviolet protection indexes between Artisto TT 10 and Vasto TT 10 in which 10% of the organic absorbent was applied to the Talc-$TiO_2$ based material, the SPF of Artisto TT 10 was 45.46, whereas the SPF of Vasto TT 10 was 114.85, showing the difference of more than 2 times. Furthermore, in the case of UVA PF, Artisto TT 10 was 18.22 and Vasto TT 10 was 123.48, showing the difference of more than 6 times between the two values. This is because the synergy effect of the ultraviolet protection was much more superior for the combination of $TiO_2$ and BMDBM than for the combination of ZnO and OMC. The same tendency was observed when the same concentration of OMC and BMDBM was coated on the Sericite-$TiO_2$ base material, thereby confirming that BMDBM has a much more superior UV blocking ability than OMC.

**Experimental Example 11. Quantitative analysis of functional ingredients of organic-inorganic coating powders**

A quantitative analysis of the UV organic absorbent coated on the organic-inorganic coating powder of the present invention was carried out. Since the OMC and BMDBM applied to the coating must be well bound to the powder without being lost during the coating process in order to have an excellent ultraviolet protection ability even when applied as a formulation, and thus the analysis was performed to confirm whether the UV absorbent was properly coated on each base material. This analysis was carried out in accordance with the method described in the analysis of "ultraviolet blocking agent" in <Guideline for the Method of Analysis of Compounding Limit Components in Cosmetics (June 06, 2010) > by HPLC (model: HP 1050 Series)

[Table 10]

| Organic-Inorganic Coating Powder | Concentration of UV Organic Absorbent (wt %) | |
| --- | --- | --- |
| | OMC | BMDBD |
| Artisto TT05 | 5.60 | - |
| Artisto TT10 | 12.58 | - |
| Artisto TT15 | 15.27 | - |
| Artisto TT20 | 14.22 | - |
| Artisto ST05 | 5.37 | - |

(continued)

| Organic-Inorganic Coating Powder | Concentration of UV Organic Absorbent (wt %) | |
| --- | --- | --- |
| | OMC | BMDBD |
| Artisto ST10 | 12.84 | - |
| Artisto ST15 | 15.52 | - |
| Artisto ST20 | 14.61 | - |
| Artisto TZ05 | 5.76 | - |
| Artisto TZ10 | 12.80 | - |
| Artisto TZ15 | 16.42 | - |
| Artisto TZ20 | 15.34 | - |
| Artisto SZ05 | 5.69 | - |
| Artisto SZ10 | 13.49 | - |
| Artisto SZ15 | 16.45 | - |
| Artisto SZ20 | 15.49 | - |
| Vasto TT02 | - | 2.19 |
| Vasto TT04 | - | 3.97 |
| Vasto TT06 | - | 6.07 |
| Vasto TT08 | - | 8.67 |
| Vasto TT10 | - | 10.05 |
| Vasto ST02 | - | 2.36 |
| Vasto ST04 | - | 4.16 |
| Vasto ST06 | - | 6.28 |
| Vasto ST08 | - | 8.69 |
| Vasto ST10 | - | 10.08 |
| Vasto TZ02 | - | 2.91 |
| Vasto TZ04 | - | 4.71 |
| Vasto TZ06 | - | 6.83 |
| Vasto TZ08 | - | 8.75 |
| Vasto TZ10 | - | 10.89 |
| Vasto SZ02 | - | 2.67 |
| Vasto SZ04 | - | 4.49 |
| Vasto SZ06 | - | 7.11 |
| Vasto SZ08 | - | 9.62 |
| Vasto SZ10 | - | 11.37 |

As shown in Table 10, in the case of OMC coated power, OMC was quantified in proportion to the concentration applied to the coating to a concentration of 5 to 15%, but at 20% concentration it rather decreased, which showed a similar result to that of the 15% OMC coated powder. On the contrary, in the case of BMDBM coated powder, it was confirmed that the BMDBM coated powder was quantified in proportion to the concentration of 2 to 10% BMDBM applied to the coating even though the concentration of organic matter used in the coating was higher than that of the organic matter applied to the OMC coated powder. As a result, in the case of the UV inorganic absorbent for the base material, it was confirmed that there existed a critical concentration at which the absorbent can be coated on the powder apart from the concentration of the organic matter applied to the coating. In addition, when comparing the degree of coating between the base materials, the coating concentration of UV organic absorbent was higher in the case of ZnO base material compared to $TiO_2$ base material.

**Example 3. Selection of optimum coating concentration of UV absorbent**

Based on the results of the experiments up until now, the optimal coating concentrations of OMC and BMDBM to be applied to powder cosmetics such as loose powder and two way cake were determined as follows.

[Table 11]

| Optimum coating concentration of UV absorbent to be applied to formulations (wt%) | | | | | |
|---|---|---|---|---|---|
| | Quantification of organic matter | UV blocking index | Quantification of UV organic absorbent | Skin sensory evaluation | Total |
| OMC optimal concentration | 10 | 15 | 15 | 10 | 10 |
| BMDBM optimal concentration | 8 | 8 | 8 | 6 | 8 |

As shown in Table 11, in the case of OMC coated powder, 15% was suitable from the results of the UV blocking index and organic matter quantitative analysis. However, from the skin function evaluation, 15% OMC was not suitable for the application to the formulation as the agglomeration between the powder became more intense which decreased applicability. In addition, from the results of all other criteria such as the quantification of organic matters and skin function evaluation, etc., the OMC concentration to be applied to the formulation was determined to be 10%.

In the case of BMDBM coated powder, 6% was suitable from the skin function evaluation, but this was not a concentration that could have a decisive effect on the ultraviolet protection ability when applied to cosmetic formulations. From the results of other criteria such as the quantification of organic matter and analysis of ultraviolet protection index, the suitable coating concentration of BMDBM to be applied to the formulation was 8%.

Based on this, the coating concentration of the UV absorbent to be applied to the formulation was determined to be 10% for OMC and 8% for BMDBM, and they were applied to powder formulations such as loose powder and two way cake.

**Experimental Example 12. Experiment of skin irritation of organic-inorganic coating powders**

Even if the UV blocking function of the organic-inorganic coating powders of the present invention was optimized, it would be difficult to apply the powders as products when they were accompanied by skin irritation, and thus, the safety of the present coating powders to skin was confirmed. The hazard evaluation was carried out by a domestic clinical evaluation agency "Derma Pro", and a skin irritation test was carried out in accordance with the evaluation schedule planned by the agency.

[Table 12]

| No | Name of coating powder | Description of coating powder |
|---|---|---|
| 1 | Fit TT 30 | Primary coating powder in which 30% of nano $TiO_2$ is coated on talc |
| 2 | Fit ST 30 | Primary coating powder in which 30% of nano $TiO_2$ is coated on Sericite |
| 3 | Liposome TT | Secondary coating powder in which hydrogenated lecithin is coated on Fit TT 30 |
| 4 | Liposome ST | Secondary coating powder in which hydrogenated lecithin is coated on Fit ST 30 |
| 5 | Artisto TT 10 | Tertiary coating powder in which 10% OMC and silicone oil for surface treatment are coated on liposome TT |
| 6 | Artisto ST 10 | Tertiary coating powder in which 10% OMC and silicone oil for surface treatment are coated on liposome ST |

(continued)

| No | Name of coating powder | Description of coating powder |
|---|---|---|
| 7 | Vasto TT 10 | Tertiary coating powder in which 10% BMDBM and silicone oil for surface treatment are coated on liposome TT |
| 8 | Vasto ST 10 | Tertiary coating powder in which 10% BMDBM and silicone oil for surface treatment are coated on liposome ST |

Total of 8 coating powders were subjected to the present skin irritation test which are shown in Table 12. The coating powders that required irritation tests were Nos. 5 to 8. However, since these powders are composite powders that have undergone various tertiary coating processes, so in the case of accompanying the irritation, it was difficult to confirm which process has accompanied the irritation. Thus, the skin irritation test was conducted even on the composite powders derived from the final coating powder development process including Nos. 1 to 4 coating base materials.

Nos. 1 and 2, which are the base materials for UV blocking double coating powder, are inorganic-inorganic coating powders. Since the $TiO_2$ coated thereon can also cause irritation, an irritation was performed thereon. Nos. 3 and 4, which are composite powders in which only hydrogenated lecithin was coated on Fit TT 30 and Fit ST 30, are secondary coating powders which are not separately surface-treated with silicone oil. The reason for carrying out this experiment on these powders is to confirm whether the ingredients of hydrogenated lecithin also accompany irritation. Hydrogenated lecithin is a key ingredient for the liposome process which encapsulates OMC and BMDBM, and it is difficult to substitute with other materials. Therefore, it was necessary to confirm whether the powder coated with hydrogenated lecithin alone causes irritation.

The coating powders Nos. 5 to 8, which are tertiary coating powder obtained by encapsulating 10% OMC and BMDBM and coating them on the base material, and then surface treating the powder with silicone oil (triethoxycaprylylsilane), are the composite powders through which the skin irritation was to be ultimately confirmed in this experiment. The reason for selecting 10% BMDBM coated powder instead of 8% BMDBM coated powder for skin irritation test was that a higher concentration was applied to compensate for a loss that would occur during the preparation of 8% BMDBM coated powder, and thus 10% BMDBM coated powder was selected for the skin irritation test as its concentration nearly approached 10%. When the 10% BMDBM coated powder was not accompanied by skin irritation, the BMDBM composite powder coated at a lower concentration was regarded as being not accompanied by skin irritation.

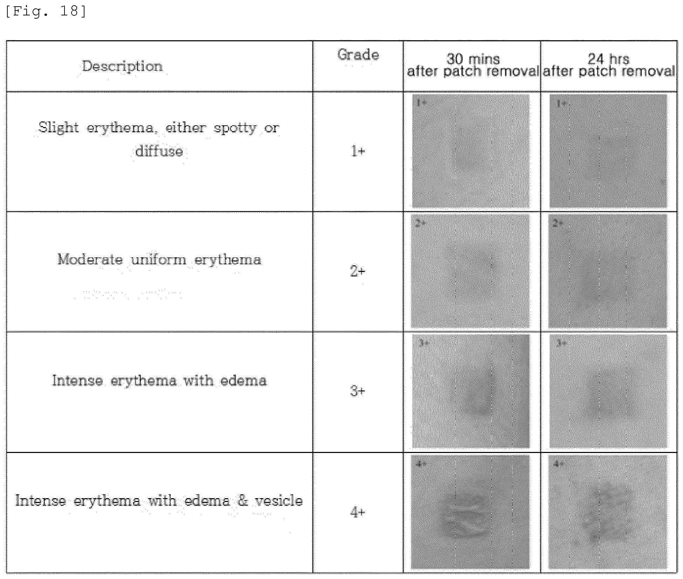

The skin irritation test was conducted by selecting 30 healthy women (mean age: 45 ± 2.9 years) who had no history of allergic contact dermatitis as subjects, and all 8 samples for this test were powder type, all of which were mixed in a ratio of 1: 1 with squalene, stirred well, filtered, and applied to the test. The degree of skin irritation and the evaluation criteria were classified into four types as shown in FIG. 18, and the eight samples were used to confirm the degree of irritation in accordance with the skin irritation evaluation by observing the skin after 30 minutes, 48 hours, and 72 hours after skin irritation. In view of the skin irritation evaluation criteria, a numerical value corresponding to "Response" was obtained through the following Equation 1, and the numerical value (R) is described in Table 13 below.

[Equation 1]

$$Response = \frac{\sum(Grade \times No. of Responders)}{4 \times n} \times 100$$

- ▪ Response: Numerical criteria of final irritation

- ▪ Grade: Irritation index

- ▪ No. of Responders: Number of subjects with irritation

- ▪ $\Sigma$: Maximum irritation index

- ▪ n: Number of all subjects participating in the test

[Table 13]

| Description of numerical criteria of final irritation | |
|---|---|
| Range of Response | Criteria |
| $0.00 \leq R < 0.87$ | Slight |
| $0.87 \leq R < 2.42$ | Mild |
| $2.42 \leq R < 3.44$ | Moderate |
| $3.44 \leq R$ | Severe |

[Table 14]

| No | Test Material | No. of Responder | 48 hrs | | | | 72 hrs | | | | Reaction Grade | | |
|---|---|---|---|---|---|---|---|---|---|---|---|---|---|
| | | | 1+ | 2+ | 3+ | 4+ | 1+ | 2+ | 3+ | 4+ | 48h | 72h | Mean |
| 01 | Fit TT 30 | 0 | - | - | - | - | - | - | - | - | 0.0 | 0.0 | 0.0 |
| 02 | Fit ST 30 | 0 | - | - | - | - | - | - | - | - | 0.0 | 0.0 | 0.0 |
| 03 | Liposome TT | 0 | - | - | - | - | - | - | - | - | 0.0 | 0.0 | 0.0 |
| 04 | Liposome ST | 1 | - | - | - | - | 1 | - | - | - | 0.0 | 0.8 | 0.4 |
| 05 | Artisto TT 10 | 0 | - | - | - | - | - | - | - | - | 0.0 | 0.0 | 0.0 |
| 06 | Artisto ST 10 | 0 | - | - | - | - | - | - | - | - | 0.0 | 0.0 | 0.0 |
| 07 | Vasto TT 10 | 0 | - | - | - | - | - | - | - | - | 0.0 | 0.0 | 0.0 |
| 08 | Vasto ST 10 | 0 | - | - | - | - | - | - | - | - | 0.0 | 0.0 | 0.0 |

As shown in Table 14, as a result of the skin irritation of the coating powders of the present invention, 1+ skin irritation was confirmed in only one powder of Liposome ST which was very light erythema. This only occurred in one of the 30 subjects at 72 hours after the removal of the powder-supported patches, and thus it is hardly a universal irritation reaction. In contrast, the organic-inorganic composite powders of the present invention coated with each 10% OMC and BMDBM, which are known to have a high possibility of skin irritation, did not cause skin irritation in any subject, thereby confirming that the coating powder groups of the present invention are safe cosmetic materials which do not accompany skin irritation even when applied to cosmetics including powder formulations.

**Example 4. Preparation of powder cosmetic applying organic-inorganic coating powder of the present invention**

The organic-inorganic coating powders of the present invention were found to have no skin irritation and an excellent ultraviolet protection effect via the experiments above. The organic-inorganic coating powder of the present invention was added to powder cosmetics as a cosmetic material in a mixing ratio shown in Tables 15 and 16 to prepare UV blocking functional loose powder and two way cake.

Preparation Example 1. Multicut loose powder

[Table 15]

| Ingredient | INCI Name | Mixing ratio (wt%) |
|---|---|---|
| Artisto TT 10 | Talc, Titanium Dioxide, Ethylhexyl Methoxycinnamate, Hydrogenated Lecithin, Triethoxycaprylylsilane, Tocopheryl Acetate, Butylene Glycol | 10.00 |
| Artisto ST 10 | Mica, Titanium Dioxide, Ethylhexyl Methoxycinnamate, Hydrogenated Lecithin, Triethoxycaprylylsilane, Tocopheryl Acetate, Butylene Glycol | 10.00 |

(continued)

| Ingredient | INCI Name | Mixing ratio (wt%) |
|---|---|---|
| Artisto TZ 10 | Talc, Zinc Oxide, Ethyhexyl Methoxycinnamate, Hydrogenated Lecithin, PEG-26-PPG-30 Phosphate and DEA, Triethoxycaprylylsilane, Tocopheryl Acetate, Butvlene Glycol | 10.00 |
| Fit SZ 30 (N) | "Mica, Zinc Oxide, PEG-26-PPG-30 Phosphate (and) DEA, Triethoxycaprylylsilane | 9.00 |
| Fit ST 30 (N) | Mica, Titanium Dioxide, Triethoxycaprylylsilane | 9.00 |
| Fit TT 30 (N) | Talc, Titanium Dioxide, Triethoxycaprylylsilane | 13.00 |
| Talc L MS | Talc, Magnesium Stearate | 15.00 |
| Silica 67 SDM | Silica, Dimethicone | 10.00 |
| - | Methyl Methacrylate Crosspolymer | 6.00 |
| TiO$_2$ PFC OTS | Titanium Dioxide, Triethoxycaprylylsilane | 7.00 |
| IOY-OTS | Iron Oxides (CI77492), Synthetic Fluorphlogopite, Triethoxycaprylylsilane | 0.70 |
| IOR-OTS | Iron Oxides (CI77491), Synthetic Fluorphlogopite, Triethoxycaprylylsilane | 0.24 |
| IOB-OTS | IronOxides (CI77499), Synthetic Fluorphlogopite, Triethoxycaprylylsilane | 0.06 |
| Total | | 100.00 |

Preparation Example 2. Muticut two way cake

[Table 16]

| Ingredient | INCI Name | Mixing ratio (%) |
|---|---|---|
| Artisto TT 10 | Talc, Titanium Dioxide, Ethylhexyl Methoxycinnamate, Hydrogenated Lecithin, Triethoxycaprylylsilane, Tocopheryl Acetate, Butylene Glycol | 10.00 |
| Artisto ST 10 | Mica, Titanium Dioxide, Ethylhexyl Methoxycinnamate, Hydrogenated Lecithin, Triethoxycaprylylsilane, TocopherylAcetate, Butylene Glycol | 10.0 |
| Vasto ST 08 | Mica, Titanium Dioxide, C12-15 Alkyl Benzoate (and) Dipropylene Glycol Dibenzoate (and) PPG-15 Sterayl Ether Dibenzoate, Butyl Methoxydibenzoylmethane, Hydrogenated Lecithin, Triethoxycaprylylsilane, Tocopheryl Acetate | 10.00 |
| Artisto TZ 10 | Talc, Zinc Oxide, Ethyhexyl Methoxycinnamate, Hydrogenated Lecithin, PEG-26-PPG-30 Phosphate (and) DEA, Triethoxycaprylylsilane, Tocopheryl Acetate, Butylene Glycol | 5.00 |
| Fit SZ 30 (N) | Mica, Zinc Oxide, PEG-26-PPG-30 Phosphate (and) DEA, Triethoxycaprylylsilane | 14.00 |
| Fit TT 30 (N) | Talc, Titanium Dioxide, Triethoxycapyrylylsilane | 18.00 |
| Talc SJ J1 OTS | Talc, Triethoxycaprylylsilane | 7.25 |
| Sericite ASP SDM | Mica, Dimethicone | 10.00 |
| - | Methyl Methacrylate Crosspolymer | 2.00 |

(continued)

| Ingredient | INCI Name | Mixing ratio (%) |
|---|---|---|
| Silica 67 SDM | Silica, Dimethicone | 5.00 |
| TiO$_2$ PFC OTS | Titanium Dioxide, Triethoxycaprylylsilane | 7.00 |
| IOY-OTS | IronOxides (CI77492), Synthetic Fluorphlogopite, Triethoxycaprylylsilane | 0.80 |
| IOR-OTS | IronOxides (CI77491), Synthetic Fluorphlogopite, Triethoxycaprylylsilane | 0.24 |
| IOB-OTS | IronOxides (CI77499), SyntheticFluorphlogopite, Triethoxycaprylylsilane | 0.06 |
| - | Caprylylic/Capric Triglycerides | 0.25 |
| - | Hexyl Laurate | 0.40 |
| Total | | 100.00 |

**Experimental Example 13. Confirmation on ultraviolet protection ability of powder cosmetic containing the organic-inorganic coating powder of the present invention**

The skin irritation test, quantification of effective ingredients, and ultraviolet protection function of UV powder cosmetics of Preparation 1 and Preparation 2 were evaluated.

Skin Irritation Test

To confirm whether the ultraviolet powder cosmetics of Preparation 1 and Preparation 2 cause skin irritation, 32 subjects were subjected to a skin irritation test in the same manner as in Experimental Example 12 above. This experiment was carried by Derma Pro, a clinical analysis agency.

[Table 17]

| No | Test Material | No. of Responder | 48 hrs | | | | 72 hrs | | | | Reaction Grade | | |
|---|---|---|---|---|---|---|---|---|---|---|---|---|---|
| | | | 1+ | 2+ | 3+ | 4+ | 1+ | 2+ | 3+ | 4+ | 48h | 72h | Mean |
| 01 | Multicut loose powder | 0 | - | - | - | - | - | - | - | - | 0.0 | 0.0 | 0.0 |
| 02 | Multicut two way cake | 0 | - | - | - | - | - | - | - | - | 0.0 | 0.0 | 0.0 |
| 03 | Negative control | 0 | - | - | - | - | - | - | - | - | 0.0 | 0.0 | 0.0 |

As shown in Table 17, it was experimentally shown that the ultraviolet powder cosmetics of Preparation Example 1 and Preparation Example 2 did not cause skin irritation at all.

Quantification of functional ingredients in powder products

The contents of the UV inorganic blocking agent and the UV organic absorbent contained in the UV powder cosmetics of Preparation Example 1 and Preparation Example 2 were analyzed by Soonchunhyang University's Industry-Academy Collaboration Corporation which is an official analysis organization of cosmetics designated by Ministry of Food and Drug Safety.

[Table 18]

|  | TiO$_2$ (%) | ZnO (%) | Sum of inorganic UV blocking agent (%) | OMC (%) | BMDBM (%) | Sum of organic absorbent (%) |
|---|---|---|---|---|---|---|
| Multicut loose powder | 17.83 | 4.95 | 22.78 | 2.98 |  | 2.98 |
| Multicut two way cake | 18.83 | 5.09 | 23.92 | 2.47 | 0.62 | 3.09 |

As shown in Table 19, the total content of the UV inorganic absorbent and the UV blocking agent in the UV powder cosmetic formulations of Preparation 1 and Preparation 2 was similar.

Evaluation of UV blocking function

Whether the UV powder cosmetics of Preparation Example 1 and Preparation Example 2 of the present invention had an ultraviolet protection function of SPF50, PA +++ or higher, which is the object of the present invention, was confirmed through the following experiment. The target value of SPF, which is a protection index for the ultraviolet B region, was 50 and the target value for PA was +++ (For reference, PA refers to a UVA-PF value converted to a "+" sign. If the UVA PF is measured to be 8 or above, PA is marked as "+++")

1) Non-clinical Evaluation

The SPF and UVA PF values for the ultraviolet powder cosmetics of Preparation Example 1 and Preparation Example 2 were confirmed by an ultraviolet protection index analysis device (SPF 290S) .

[Table 19]

|  | SPF | UVA PF | Critical wavelength |
|---|---|---|---|
| Multicut loose powder | 138.35 | 59.95 | 380.14 |
| Multicut two way cake | 198.03 | 95.20 | 381.74 |

As shown in Table 20, the mean values of SPF and UVA PF were 138.35 and 59.95 for multicut loose powder of Preparation Example 1 and 198.03 and 95.02 for multicut two way cake of Preparation Example 2, respectively.

2) Clinical Evaluation

The ultraviolet protection function of the UV powder cosmetics of Preparation Example 1 and Preparation Example 2 were carried out by Derma Pro, a clinical evaluation agency to confirm the SPF and UVA PE.
First, in the SPF test, the ultraviolet protection index for each product group was measured for 10 subjects without skin disease by the Japan Cosmetic Industry Association UV blocking index (SPF) measurement method (JCIA, 2011). In addition, this test was conducted by irradiating UV-B (290 to 320 nm) on the subjects' back to measure the minimum erythema dose (MED) for the standard sample, sample for measurement and the non-coated area (skin on which no sample was coated). The determination of the minimum erythema dose was carried out after 16 to 24 hours from the irradiation of ultraviolet rays by two skilled examiners according to the erythema determination criteria. The criteria are as shown in Table 20 below, and the ultraviolet B protection index was calculated as shown in Equation 2 below.

[Table 20]

| Evaluation | Description | Note |
|---|---|---|
| - | No reaction |  |
| + | Slight erythema reaction is seen, but it is not sufficient to be determined as the minimum erythema dose or when erythema occurs in less than 50% of the area irradiated with ultraviolet rays or when the erythematous form of the prototype is not complete |  |

(continued)

| Evaluation | Description | Note |
|---|---|---|
| + | Erythema is clearly visible compared to the area not irradiated with ultraviolet rays and occupies the entire area of the irradiated area of the ultraviolet rays, and the shape of the prototype (2/3) is completely visible | Minimum erythema dose (MED) |
| ++ | Erythema reaction and swelling of skin surface are clearly seen in 100% of ultraviolet irradiated area | |

[Equation 2]

UV-blocking index by subject (SPF)= Minimum erythema dose (MED) of the coated area / Minimum erythema dose (MED) of the non-coated area

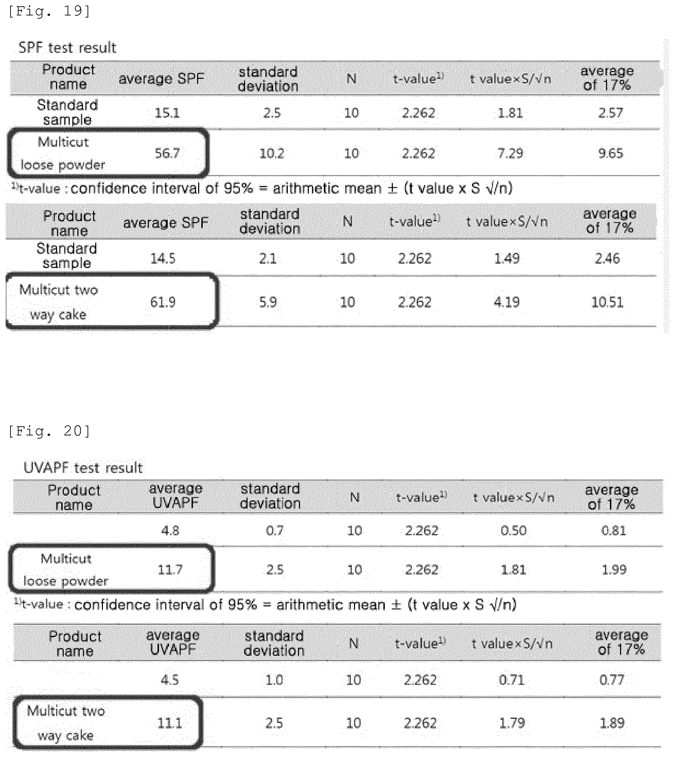

As shown in FIG. 19, the SPF blocking indexes were 56.7 for multicut loose powder of Preparation Example 1 and 61.9 for multicut two way cake of Preparation Example 2.

Next, the UVA PF test was carried out in the same manner as the SPF test. The difference was that the minimum persistent pigment darkening dose (MPPDD) was measured by irradiating ultraviolet rays of 320 to 400 nm range to the subjects' back. The measurement of darkening was evaluated by two skilled examiners after 2 to 24 hours of UV irradiation, and the criteria for determining darkening are shown in Table 21. According to the MPPDD determined by the criteria, the ultraviolet protection index (UVA PFi) of each subject was calculated according to Equation 3 below.

[Table 21]

| Evaluation | Description | Note |
|---|---|---|
| - | No reaction | |
| ± | Slight darkening reaction is seen, but it is not sufficient to be determined as the minimum persistent pigment darkening dose or when darkening occurs in less than 50% of the area irradiated with ultraviolet rays or when the darkening form of the prototype is not complete | |
| + | Darkening reaction is clearly visible compared to the area not irradiated with ultraviolet rays and occupies the entire area of the | Minimum persistent pigment darkening dose |
| | irradiated area of the ultraviolet rays, and the shape of the prototype (2/3) is completely visible | (MPPDD) |

[Equation 3]

UV-blocking index by subject (UVA PF)= Minimum persistent pigment darkening dose (MPPDD) of the coated area / Minimum persistent pigment darkening dose (MPPDD) of the non-coated area

As shown in FIG. 20, the UVA PF protection indexes were 11.7 for multicut loose powder of Preparation Example 1 and 11.1 for multicut two way cake of Preparation Example 2.

As a result, it was confirmed that the powder cosmetics containing the organic-inorganic composite powder of the present invention has an ultraviolet protection function of SPF 50, PA +++ or higher.

**[INDUSTRIAL APPLICABILITY]**

The present invention is an extremely useful invention in the functional cosmetics industry since it has an excellent effect of maximizing ultraviolet protection ability while reducing skin irritation.

**Claims**

1. Method for the preparation of a UV blocking functional composite powder consisting of an inorganic powder as a coating base material with a primary coating of an inorganic UV blocking agent to yield a so-called inorganic-inorganic coating powder, a secondary coating of an UV organic absorbent encapsulated into a liposome, and a tertiary coating of a silicone oil wherein the method comprises the following steps:

   (a) preparing an inorganic-inorganic coating powder by primarily coating talc or sericite with an inorganic UV blocking agent selected from titanium dioxide and zinc oxide;
   (b) separately preparing an UV absorbent sol by encapsulating ethylhexyl methoxycinnamte or butyl methoxydibenzoylmethane in hydrogenated lecithin;
   (c) preparing an organic-inorganic coating powder by secondarily coating the inorganic-inorganic powder prepared in step (a) as a coating base with the UV absorbent sol of step (b); followed by
   (d) tertiarily coating the powder with triethoxycaprylylsilane.

2. The method according to claim 1, wherein the titanium dioxide and zinc oxide have a particle size of 20 to 50 nm and a concentration of 30% by weight.

3. The method according to any of the above claims, wherein the inorganic-inorganic coating powder is prepared to a method comprising: (a) heating 500 to 800% by weight of purified water based on the total composition of inorganic-inorganic coating powder to 75 to 80°C, then adding thereto 70 to 90% by weight of inorganic powder, followed by stirring, mixing and dispersing the mixture, and then adjusting the heating rate to precipitate the inorganic powder below purified water; b) separately, preparing a solution containing 10 to 30% by weight of the UV inorganic blocking agent, and coating the same by adding and stirring; c) mixing, stirring and coating the inorganic powder of step (a) and the solution of step (b), then allowing the mixture to stand for 12 hours or more and cooling to confirm the phase separation and the transparency of the supernatant, followed by drying the resultant at 105 to 110°C for 15 hours or more to make the water content less than 1% by dehydration; d) pulverizing and filtering the dried product obtained in step (c) to make the particle size uniform.

4. The method according to any of the above claims, wherein the coating composition comprising the UV organic absorbent is an ethylhexyl methoxycinnamate sol, produced by a method comprising: (a) heating 500 to 900% by weight of purified water based on the total composition of ethylhexyl methoxycinnamate sol to 70 to 80°C, then adding thereto 1 to 5% by weight of butylene glycol, followed by stirring, mixing and dispersing the mixture; (b) adding and dispersing 1 to 10% by weight of hydrogenated lecithin at the same temperature as step (a), followed by adding, dispersing and liposomizing 5 to 20% by weight of ethylhexyl methoxycinnamate; (c) cooling the product obtained in step (a) and the product obtained in step (b) to 30 to 40°C, and treating the products with an ultra-high pressure emulsifier.

5. The method according to any of claims 1 to 4, wherein the coating composition comprising the UV organic absorbent is a butyl methoxydibenzoylmethane sol, produced by a method comprising: (a) heating 500 to 1000% by weight of purified water based on the total composition of butyl methoxydibenzoylmethane sol to 70 to 80°C and then adding thereto 1 to 10% by weight of hydrogenated lecithin, followed by uniformly stirring and dispersing the mixture; (b) separately, heating a mixture of 3 to 15% by weight of $C_{12-15}$ alkylbenzoate, dipropylene glycol dibenzoate and PPG-15 stearyl ether benzoate to 60 to 70°C and then adding, dispersing and dissolving 2 to 10% by weight of butyl methoxydibenzoylmethane; (c) adding 0.1 to 1.0% by weight of tocopheryl acetate to the product obtained in step (a), then adding, dispersing and liposomizing the mixture obtained in step (b).

6. The method according to any of the above claims, the method comprising the following steps: (a) heating 500 to 1000% by weight of purified water based on the total composition of organic-inorganic composite powder to 70 to 80°C and then adding thereto 70 to 95% by weight of inorganic-inorganic powder of claim 3, as a coating base material, followed by mixing and dispersing the mixture: (b) adding 0.5 to 2.0% by weight of anhydrous magnesium sulfate and then dispersing and dissolving the same; (c) preparing a coating solution by adding and coating any one of ethylhexyl methoxycinnamate sol (Sol) of claim 4 or butyl methoxydibenzoylmethane sol (11) of claim 5; (d) confirming the phase-separation and transparency of the supernatant after allowing the coating solution obtained in step (c) to stand for more than 12 hours and cooling, (e) filtering and dehydrating the coating solution obtained in step (d) at room temperature and drying at 70 to 80°C for at least 15 hours to adjust the water content to 2% or less; (f) pulverizing the dried product obtained in the drying step and tertiarily the dried product, as a coating base material by adding 1 to 5% by weight of triethoxycaprylylsilane; (g) drying the coating product obtained in step (f) at 70 to 80°C for at least 12 hours to adjust the water content to 2% or less; and (h) pulverizing, filtering and packaging the dried product obtained in the drying step

7. The method of claim 6, wherein the coating concentration of the ethylhexyl methoxycinnamate in step (c) is 5 to 20% by weight.

8. The method of claim 6, wherein the coating concentration of the butyl methoxydibenzoylmethane in step (c) is 2 to 10% by weight.

9. The method of claim 6, wherein the coating of triethoxycaprylylsilane in step (f) is carried out through a drying process at 70 to 80°C.

10. An UV blocking functional composite coating powder prepared according to the method of any of claims 1 to 9.

11. An UV blocking cosmetic composition prepared according to the method of any of claims 1 to 9.


**Patentansprüche**

1. Verfahren zur Herstellung eines UV-blockierenden funktionellen Verbundstoffpulvers, das aus einem anorganischen Pulver als Beschichtungsgrundmaterial mit einer primären Beschichtung aus einem anorganischen UV-Blockierungsmittel unter Bildung eines sogenannten Anorganisch-Anorganisch-Beschichtungspulvers, einer sekundären Beschichtung aus einem organischen UV-Absorber, der in einem Liposom eingekapselt ist, und einer tertiären Beschichtung aus einem Silikonöl besteht, wobei das Verfahren die folgenden Schritte umfasst:

(a) Herstellen eines Anorganisch-Anorganisch-Beschichtungspulvers durch primäres Beschichten von Talk oder Sericit mit einem anorganischen UV-Blockierungsmittel, das aus Titandioxid und Zinkoxid ausgewählt ist;
(b) separates Herstellen eines UV-absorbierenden Sols durch Einkapseln von Ethylhexylmethoxycinnamat oder Butylmethoxydibenzoylmethan in hydriertem Lecithin;
(c) Herstellen eines Organisch-Anorganisch-Beschichtungspulvers durch sekundäres Beschichten durch sekundäres Beschichten des in Schritt (a) hergestellten Anorganisch-Anorganisch-Pulvers als Beschichtungsbasis mit dem UV-absorbierenden Sol aus Schritt (b); und anschließend
(d) tertiäres Beschichten des Pulvers mit Triethoxycaprylylsilan.

2. Verfahren gemäß Anspruch 1, wobei das Titandioxid und das Zinkoxid eine Teilchengröße von 20 bis 50 nm und eine Konzentration von 30 Gew.-% aufweisen.

3. Verfahren gemäß einem der vorstehenden Ansprüche, wobei das Anorganisch-Anorganisch-Beschichtungspulver nach einem Verfahren hergestellt wird, das Folgendes umfasst: (a) Erhitzen von 500 bis 800 Gew.-% gereinigtem Wasser, bezogen auf die Gesamtzusammensetzung des Anorganisch-Anorganisch-Beschichtungspulvers, auf 75 bis 80 °C, dann Hinzufügen von 70 bis 90 Gew.-% anorganisches Pulver, anschließend Rühren, Mischen und Dispergieren des Gemischs und dann Einstellen der Aufheizgeschwindigkeit, um das anorganische Pulver unter gereinigtem Wasser auszufällen; b) separates Herstellen einer Lösung, die 10 bis 30 Gew.-% des anorganischen UV-Blockierungsmittels enthält, und Auftragen derselben durch Hinzufügen und Rühren; c) Mischen, Rühren und Beschichten des anorganischen Pulvers aus Schritt (a) mit der Lösung aus Schritt (b), dann Stehenlassen des Gemischs während 12 Stunden oder mehr und Abkühlen, um die Phasentrennung und die Transparenz des Überstands zu bestätigen, anschließend Trocknen des Produkts bei 105 bis 110 °C während 15 Stunden oder mehr, so dass der Wassergehalt weniger als 1% beträgt, durch Dehydratisierung; d) Pulverisieren und Filtrieren des in Schritt (c) erhaltenen getrockneten Produkts, so dass die Teilchengröße gleichmäßig wird.

4. Verfahren gemäß einem der vorstehenden Ansprüche, wobei die Beschichtungszusammensetzung, die den organischen UV-Absorber umfasst, ein Ethylhexylmethoxycinnamat-Sol ist, das nach einem Verfahren hergestellt wird, das Folgendes umfasst: (a) Erhitzen von 500 bis 900 Gew.-% gereinigtem Wasser, bezogen auf die Gesamtzusammensetzung des Ethylhexylmethoxycinnamat-Sols, auf 70 bis 80 °C, dann Hinzufügen von 1 bis 5 Gew.-% Butylenglycol, anschließend Rühren, Mischen und Dispergieren des Gemischs; (b) Hinzufügen und Dispergieren von 1 bis 10 Gew.-% hydriertes Lecithin bei derselben Temperatur wie in Schritt (a), anschließend Hinzufügen, Dispergieren und Liposomisieren von 5 bis 20 Gew.-% Ethylhexylmethoxycinnamat; (c) Abkühlen des in Schritt (a) erhaltenen Produkts und des in Schritt (b) erhaltenen Produkts auf 30 bis 40 °C und behandeln der Produkte mit einem Ultrahochdruckemulgator.

5. Verfahren gemäß einem der Ansprüche 1 bis 4, wobei die Beschichtungszusammensetzung, die den organischen UV-Absorber umfasst, ein Butylmethoxydibenzoylmethan-Sol ist, das nach einem Verfahren hergestellt wird, das Folgendes umfasst: (a) Erhitzen von 500 bis 1000 Gew.-% gereinigtem Wasser, bezogen auf die Gesamtzusammensetzung des Butylmethoxydibenzoylmethan-Sols, auf 70 bis 80 °C und dann Hinzufügen von 1 bis 10 Gew.-% hydriertes Lecithin, anschließend gleichmäßiges Rühren und Dispergieren des Gemischs; (b) separates Erhitzen eines Gemischs von 3 bis 15 Gew.-% $C_{12-15}$-Alkylbenzoat, Dipropylenglycoldibenzoat und PPG-15-Stearyletherbenzoat auf 60 bis 70 °C und dann Hinzufügen, Dispergieren und Auflösen von 2 bis 10 Gew.-% Butylmethoxydibenzoylmethan; (c) Hinzufügen von 0,1 bis 1,0 Gew.-% Tocopherylacetat zu dem in Schritt (a) erhaltenen Produkt, dann Hinzufügen, Dispergieren und Liposomisieren des in Schritt (b) erhaltenen Gemischs.

6. Verfahren gemäß einem der vorstehenden Ansprüche, wobei das Verfahren die folgenden Schritte umfasst: (a) Erhitzen von 500 bis 1000 Gew.-% gereinigtem Wasser, bezogen auf die Gesamtzusammensetzung des Organisch-Anorganisch-Beschichtungspulvers, auf 70 bis 80 °C und dann Hinzufügen von 70 bis 95 Gew.-% des Anorganisch-Anorganisch-Pulvers gemäß Anspruch 3 als Beschichtungsgrundmaterial, anschließend Mischen und Dispergieren des Gemischs: (b) Hinzufügen von 0,5 bis 2,0 Gew.-% wasserfreies Magnesiumsulfat und dann Dispergieren und Auflösen desselben; (c) Herstellen einer Beschichtungslösung durch Hinzufügen und Auftragen von entweder Ethylhexylmethoxycinnamat-Sol (Sol) gemäß Anspruch 4 oder Butylmethoxydibenzoylmethan-Sol (11) gemäß Anspruch 5; (d) Bestätigen der Phasentrennung und der Transparenz des Überstands nach Stehenlassen der in Schritt (c) erhaltenen Lösung während mehr als 12 Stunden und Abkühlen, (e) Filtrieren und Dehydratisieren der in Schritt (d) erhaltenen Beschichtungslösung bei Raumtemperatur und Trocknen bei 70 bis 80 °C während wenigstens 15 Stunden, um den Wassergehalt auf 2% oder weniger einzustellen; (f) Pulverisieren des im Trocknungsschritt erhaltenen getrockneten Produkts und tertiäres Beschichten des getrockneten Produkts als Beschichtungsgrundmaterial durch Hinzufügen von 1 bis 5 Gew.-% Triethoxycaprylylsilan; (g) Trocknen des in Schritt (f) erhaltenen Beschichtungsprodukts bei 70 bis 80 °C während wenigstens 12 Stunden, um den Wassergehalt auf 2% oder weniger einzustellen; und (h) Pulverisieren, Filtrieren und Verpacken des im Trocknungsschritt erhaltenen getrockneten Produkts.

7. Verfahren gemäß Anspruch 6, wobei die Beschichtungskonzentration des Ethylhexylmethoxycinnamats in Schritt (c) 5 bis 20 Gew.-% beträgt.

8. Verfahren gemäß Anspruch 6, wobei die Beschichtungskonzentration des Butylmethoxydibenzoylmethans in Schritt (c) 2 bis 10 Gew.-% beträgt.

9. Verfahren gemäß Anspruch 6, wobei die Beschichtung von Triethoxycaprylylsilan in Schritt (f) durch ein Trocknungsverfahren bei 70 bis 80 °C durchgeführt wird.

10. UV-blockierendes funktionelles Verbundstoffbeschichtungspulver, hergestellt nach dem Verfahren gemäß einem der Ansprüche 1 bis 9.

11. UV-blockierende Kosmetikzusammensetzung, hergestellt nach dem Verfahren gemäß einem der Ansprüche 1 bis 9.

**Revendications**

1. Procédé pour la préparation d'une poudre composite fonctionnelle bloquant les UV, consistant en une poudre inorganique comme matériau de revêtement de base avec un revêtement primaire d'un agent de blocage UV inorganique pour obtenir une poudre de revêtement dite inorganique-inorganique, un revêtement secondaire d'un agent absorbant les UV organique encapsulé dans un liposome, et un revêtement tertiaire d'une huile de silicone, dans lequel ledit procédé comprend les étapes suivantes consistant à :

(a) préparer une poudre de revêtement inorganique-inorganique en enrobant primairement du talc ou de la séricite avec un agent de blocage UV inorganique choisi parmi le dioxyde de titane et l'oxyde de zinc,

(b) préparer séparément un sol absorbant les UV en encapsulant du méthoxycinnamate d'éthylhexyle ou du butylméthoxydibenzoylméthane dans de la lécithine hydrogénée,

(c) préparer une poudre de revêtement organique-inorganique en enrobant secondairement la poudre inorganique-inorganique préparée dans l'étape (a) comme couche de base avec le sol absorbant les UV de l'étape (b), et ensuite

(d) enrober tertiairement la poudre avec du triéthoxycaprylylsilane.

2. Procédé selon la revendication 1, dans lequel le dioxyde de titane et l'oxyde de zinc présentent une granulométrie allant de 20 à 50 nm, et une concentration de 30 % en poids.

3. Procédé selon l'une quelconque des revendications précédentes, dans lequel ladite poudre de revêtement inorganique-inorganique est préparée selon une méthode comprenant les étapes consistant à (a) chauffer 500 à 800 % en poids d'eau purifiée, par rapport à la composition totale de ladite poudre de revêtement inorganique-inorganique, à 75 à 80 °C, ensuite ajouter 70 à 90 % en poids de poudre inorganique, ensuite agiter, mélanger et disperser le mélange, et puis ajuster la vitesse de chauffage pour précipiter la poudre inorganique sous de l'eau purifiée, b) préparer séparément une solution contenant 10 à 30 % en poids dudit agent de blocage UV inorganique, et l'appliquer en ajoutant et agitant, c) mélanger, agiter et enrober la poudre inorganique de l'étape (a) avec la solution de l'étape (b), ensuite laisser le mélange au repos pendant 12 heures ou plus, et refroidir pour confirmer la séparation des phases et la transparence du surnageant, ensuite sécher le produit obtenu à 105 à 110 °C pendant 15 heures ou plus pour obtenir une teneur en eau de moins de 1 % par déshydratation, d) pulvériser et filtrer le produit séché obtenu dans l'étape (c) pour obtenir une granulométrie uniforme.

4. Procédé selon l'une quelconque des revendications précédentes, dans lequel la composition d'enrobage comprenant ledit agent absorbant les UV organique est un sol de méthoxycinnamate d'éthylhexyle, préparé par une méthode comprenant les étapes consistant à (a) chauffer 500 à 900 % en poids d'eau purifiée, par rapport à la composition totale dudit sol de méthoxycinnamate d'éthylhexyle, à 70 à 80 °C, ensuite ajouter 1 à 5 % en poids de butylène glycol, ensuite agiter, mélanger et disperser le mélange, (b) ajouter et disperser 1 à 10 % en poids de lécithine hydrogénée à la même température qu'à l'étape (a), ensuite ajouter, disperser et liposomiser 5 à 20% en poids de méthoxycinnamate d'éthylhexyle, (c) refroidir le produit obtenu dans l'étape (a) et le produit obtenu dans l'étape (b) à 30 à 40 °C, et traiter les produits avec un émulsifiant à ultra-haute pression.

5. Procédé selon l'une quelconque des revendications 1 à 4, dans lequel la composition d'enrobage comprenant ledit agent absorbant les UV organique est un sol de butylméthoxydibenzoylméthane, préparé par une méthode comprenant les étapes consistant à (a) chauffer 500 à 1000 % en poids d'eau purifiée, par rapport à la composition totale dudit sol de butylméthoxydibenzoylméthane, à 70 à 80 °C, ensuite ajouter 1 à 10 % en poids de lécithine hydrogénée, ensuite agiter uniformément et disperser le mélange, (b) chauffer séparément un mélange de 3 à 15 % en poids de benzoate d'alkyle en $C_{12-15}$, dibenzoate de dipropylène glycol et benzoate d'éther stéarylique de PPG-15 à 60 à 70 °C, et ensuite ajouter, disperser et dissoudre 2 à 10 % en poids, de butylméthoxydibenzoylméthane, (c) ajouter 0,1 à 1,0 % en poids d'acétate de tocophéryle au produit obtenu dans l'étape (a), ensuite ajouter, disperser et liposomiser le mélange obtenu dans l'étape (b).

6. Procédé selon l'une quelconque des revendications précédentes, ledit procédé comprenant les étapes suivantes consistant à chauffer 500 à 1000 % en poids d'eau purifiée, par rapport à la composition totale de ladite poudre composite organique-inorganique, à 70 à 80 °C, et ensuite ajouter 70 à 95 % en poids de la poudre inorganique-inorganique selon la revendication 3, comme matériau de revêtement de base, ensuite mélanger et disperser le mélange, (b) ajouter 0,5 à 2,0 % en poids de sulfate de magnésium anhydre, et ensuite le disperser et dissoudre, (c) préparer une solution de revêtement en ajoutant et appliquant soit le sol de méthoxycinnamate d'éthylhexyle (sol) selon la revendication 4 soit le sol de butylméthoxydibenzoylméthane (11) selon la revendication 5, (d) confirmer la séparation des phases et la transparence du surnageant après avoir laissé la solution de revêtement obtenue dans l'étape (c) au repos pendant plus de 12 heures, et refroidir, (e) filtrer et déshydrater la solution de revêtement obtenue dans l'étape (d) à température ambiante, et sécher à 70 à 80 °C pendant au moins 15 heures pour ajuster la teneur en eau à 2 % ou moins, (f) pulvériser le produit séché obtenu dans l'étape de séchage et enrober tertiairement le produit séché comme matériau de revêtement de base en ajoutant 1 à 5 % en poids de triéthoxycaprylylsilane, (g) sécher le produit de revêtement obtenu dans l'étape (f) à 70 à 80 °C pendant au moins 12 heures pour ajuster la teneur en eau à 2 % ou moins, et (h) pulvériser, filtrer et emballer le produit séché obtenu dans l'étape de séchage.

7. Procédé selon la revendication 6, dans lequel la concentration de revêtement dudit méthoxycinnamate d'éthylhexyle dans l'étape (c) est de 5 à 20 % en poids.

8. Procédé selon la revendication 6, dans lequel la concentration de revêtement dudit butylméthoxydibenzoylméthane dans l'étape (c) est de 2 à 10 % en poids.

9. Procédé selon la revendication 6, dans lequel l'application de triéthoxycaprylylsilane dans l'étape (f) est effectuée par un procédé de séchage à 70 à 80 °C.

10. Poudre de revêtement composite fonctionnelle bloquant les UV, préparée par le procédé selon l'une quelconque des revendications 1 à 9.

11. Composition cosmétique bloquant les UV, préparée par le procédé selon l'une quelconque des revendications 1 à 9.

[Fig. 1]

Preparation process of
inorganic-inorganic coating powder

Measuring and heating purified water
(Agi mixer, 75~80℃)

Adding inorganic powder
(talc or sericite) (Agi mixer)

Preparing 10 to 30% UV inorganic blocking
agent sol (titanium dioxide or zinc oxide)

Coating inorganic powder with UV inorganic
blocking agent sol (Agi mixer)

Allowing the resultant to stand and cool
(at least 12 hrs)

Confirming phase separation
between inorganic powder and coating solution

Dehydrating and filtering,
drying (105~110℃, at least 15 hrs)

Confirmation of Moisture content of 1% or less

Pulverizing (Atomizer)

Inorganic-inorganic coating powder

[Fig. 2]

Fit ST 10 (P)[1]      Fit ST 20 (P)[2]      Fit ST 30 (P)[3]

* 1) Sericite 90% + pigment $TiO_2$ 10% coated powder
  2) Sericite 80% + pigment $TiO_2$ 20% coated powder
  3) Sericite 70% + pigment $TiO_2$ 30% coated powder

[Fig. 3]

Fit ST 10 (N)[4]      Fit ST 20 (N)[5]      Fit ST 30 (N)[6]

* 4) Sericite 90% + nano $TiO_2$ 10% coated powder
  5) Sericite 80% + nano $TiO_2$ 20% coated powder
  6) Sericite 70% + nano $TiO_2$ 30% coated powder

[Fig. 4]

Fit SZ 10 (P)[7]          Fit SZ 17 (P)[8]          Fit SZ 30 (P)[9]

\* 7) Sericite 90% + pigment ZnO 10% coated powder
  8) Sericite 80% + pigment ZnO 17% coated powder
  9) Sericite 70% + pigment ZnO 30% coated powder

[Fig. 5]

Fit SZ 10 (N)[10]          Fit SZ 20 (N)[11]          Fit SZ 30 (N)[12]

\* 10) Sericite 90% + Nano ZnO 10% coated powder
  11) Sericite 80% + Nano ZnO 20% coated powder
  12) Sericite 70% + Nano ZnO 30% coated powder

[Fig. 6]

Fit ST 10 (P)[1]     Fit ST 20 (P)[2]     Fit ST 30 (P)[3]

* 1) Sericite 90% + pigment $TiO_2$ 10% coated powder
  2) Sericite 80% + pigment $TiO_2$ 20% coated powder
  3) Sericite 70% + pigment $TiO_2$ 30% coated powder

[Fig. 7]

Fit ST 10 (N)[4]     Fit ST 20 (N)[5]     Fit ST 30 (N)[6]

* 4) Sericite 90% + nano $TiO_2$ 10% coated powder
  5) Sericite 80% + nano $TiO_2$ 20% coated powder
  6) Sericite 70% + nano $TiO_2$ 30% coated powder

[Fig. 8]

Fit ST 10 (N)[4]          Fit ST 20 (N)[5]          Fit ST 30 (N)[6]

* 4) Sericite 90% + nano TiO$_2$ 10% coated powder
  5) Sericite 80% + nano TiO$_2$ 20% coated powder
  6) Sericite 70% + nano TiO$_2$ 30% coated powder

[Fig. 9]

Fit ST 10 (N)[4]          Fit ST 20 (N)[5]          Fit ST 30 (N)[6]

* 4) Sericite 90% + nano TiO$_2$ 10% coated powder
  5) Sericite 80% + nano TiO$_2$ 20% coated powder
  6) Sericite 70% + nano TiO$_2$ 30% coated powder

[Fig. 10]

(× 2,000 )  (× 5,000 )  (× 8,000 )

Fit ST 10 (P)

Fit ST 20 (P)

Fit ST 30 (P)

[Fig. 11]

Fit ST 10 (N)

Fit ST 20 (N)

Fit ST 30 (N)

[Fig. 12]

Fit SZ 10 (P)

Fit SZ 20 (P)

Fit SZ 30 (P)

[Fig. 13]

[Fig. 14]

## Preparation process of OMC sol

Adding and heating purified water and butylene glycol (Homo mixer, 70~80℃)

⇩

Adding and dispersing hydrogenated lecithin (Homo mixer, 70~80℃)

⇩

Adding ethylhexyl methoxycinnamate (OMC)(Homo mixer, 70~80℃)

⇩

Cooling and treating with ultra-high pressure emulsifier twice (around 30℃, Microfludizer)

⇩

Preparing ethylhexyl methoxycinnamate sol

[Fig. 15]

# Preparation process of BMDBM sol

| Adding and heating purified water and butylene glycol (Homo mixer, 70~80℃) | Heating the mixture solution of C12-C15 alkylbenzoate, dipropylene glycol dibenzoate and PPG-15 stearyl ether benzoate (60~65℃) |
|---|---|

⬇ ⬇

| Adding and dispersing hydrogenated lecithin (Homo mixer, 70~80℃) | Adding and dispersing BMDBM solution (60~65℃) |
|---|---|

Adding Tocopheryl Acetate ⬂　⬀

Adding and dispersing BMDBM solution (70~80℃, 20~30 min)

⬇

Cooling to 40°C or below

⬇

Preparing butyl methoxydibenzoylmethane sol

[Fig. 16]

Preparation process of organic-inorganic coating powder

Measuring and heating purified water
(Agi mixer, 70~80℃)

▽

Adding inorganic-inorganic coating powder

▽

Adding and stirring magnesium metal salt
(70~80℃, 10~20 min)

▽

Adding UV organic absorbent sol
(70~80℃, 20~30 min)

▽

Allowing the resultant to stand and cool
(at least 12 hrs)

▽ Confirmation of phase isolation
of powder and coating solution

Filtering and dehydrating coating solution

▽

Drying (70~80℃, at least 15 hrs)

▽

Primary pulverization (Atomizer)

▽ Coating of silicone oil
to prevent elution

Drying (70~80℃)

▽ les than 2.0% of moisture

Organic-inorganic coating powder

[Fig. 17]

[Fig. 18]

| Description | Grade | 30 mins after patch removal | 24 hrs after patch removal |
|---|---|---|---|
| Slight erythema, either spotty or diffuse | 1+ | | |
| Moderate uniform erythema | 2+ | | |
| Intense erythema with edema | 3+ | | |
| Intense erythema with edema & vesicle | 4+ | | |

[Fig. 19]

SPF test result

| Product name | average SPF | standard deviation | N | t-value[1] | t value×S/√n | average of 17% |
|---|---|---|---|---|---|---|
| Standard sample | 15.1 | 2.5 | 10 | 2.262 | 1.81 | 2.57 |
| Multicut loose powder | 56.7 | 10.2 | 10 | 2.262 | 7.29 | 9.65 |

[1]t-value : confidence interval of 95% = arithmetic mean ± (t value x S √/n)

| Product name | average SPF | standard deviation | N | t-value[1] | t value×S/√n | average of 17% |
|---|---|---|---|---|---|---|
| Standard sample | 14.5 | 2.1 | 10 | 2.262 | 1.49 | 2.46 |
| Multicut two way cake | 61.9 | 5.9 | 10 | 2.262 | 4.19 | 10.51 |

[Fig. 20]

UVAPF test result

| Product name | average UVAPF | standard deviation | N | t-value[1] | t value×S/√n | average of 17% |
|---|---|---|---|---|---|---|
|  | 4.8 | 0.7 | 10 | 2.262 | 0.50 | 0.81 |
| Multicut loose powder | 11.7 | 2.5 | 10 | 2.262 | 1.81 | 1.99 |

[1]t-value : confidence interval of 95% = arithmetic mean ± (t value x S √/n)

| Product name | average UVAPF | standard deviation | N | t-value[1] | t value×S/√n | average of 17% |
|---|---|---|---|---|---|---|
|  | 4.5 | 1.0 | 10 | 2.262 | 0.71 | 0.77 |
| Multicut two way cake | 11.1 | 2.5 | 10 | 2.262 | 1.79 | 1.89 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20130047266
- KR 100445543

- KR 100744945
- KR 101006343

**Non-patent literature cited in the description**

- **YASUO, I.** The mechanism of oryzanol activity and consideration to applicating efficiency in cosmetic. *J. Fragrance,* 1980, vol. 45, 92-97
- **SYDNEY, H. D.** Contact sensitization and photocontact sensitization of sunscreening agents. *Physician's Guide to Sunscreens,* 1981, 95-122
- Analysis report of cosmetics industry. Korea Health Industry Development Institute, 2013

- ultraviolet blocking agent. Guideline for the Method of Analysis of Compounding Limit Components in Cosmetics. 06 June 2010
- Ministry of Food and Drug Safety. Soonchunhyang University's Industry-Academy Collaboration Corporation